# EUROPEAN PATENT APPLICATION

(11) **EP 1 355 157 A1**
(43) Date of publication of application: **22.10.2003**
(21) Application number: 02292619.0
(22) Date of filing: 22.10.2002
(51) Int. Cl.: G01N 33/574, G01N 33/50

(54) **FADD proteins, phosphorylated p38-MAPK and FasL as tumours markers**

(30) Priority: 24.12.2001 EP 01403359
(71) Applicant: INSTITUT NATIONAL DE LA SANTE ET DE LA RECHERCHE MEDICALE (INSERM), 75654 Paris Cédex 13 (FR); CENTRE NATIONAL DE LA RECHERCHE SCIENTIFIQUE (CNRS), 75794 Paris Cedex 16 (FR)
(72) Inventor: Chiocchia, Gilles, 95270 Luzarches (FR); Tourneur, Léa, 75012 Paris (FR); Feunteun, Jean, 75005 Paris (FR); Michiels, Francine, 94240 l'Hay Les Roses (FR); Buzyn, Agnès, 75005 Paris (FR)
(74) Representative: Paris, Fabienne

(57) **Abstract**

The application relates to the use of at least one protein selected from the group consisting of FADD proteins and phosphorylated p38-MAPK, as a marker for absence of *in vivo* tumour. This use may further be complemented by the use of the FasL protein as a marker for presence of *in vivo* tumour. The (respective) amount(s) of FADD proteins and phosphorylated p38-MAPK indeed decrease, sometimes up to zero, with tumour development, while FasL expression is gained. FADD proteins are secreted from out of the tumour cells into their extracellular environment. A low cellular amount and a high extracellular amount of FADD proteins are prognostic of resistance to chemotherapy.

SEQUENCE LISTING

<110>  I.N.S.E.R.M. (Institut National de la Santé et de la Recherche Médicale)
      C.N.R.S (Centre National de la Recherche Scientifique)

<120>  New tumour markers and biological applications thereof

<130>  FP-CHIOCCHIA 2

<150>  EP01403359.1
<151>  2001-12-24

<160>  26

<170>  PatentIn version 3.1

<210>  1
<211>  22
<212>  DNA
<213>  Artificial Sequence

<220>
<223>  primer
<400>  1
cagcagtgcc acttcatctt gg                                                22

<210>  2
<211>  22
<212>  DNA
<213>  Artificial Sequence

<220>
<223>  primer
<400>  2
ttcactccag agatcagagc gg                                                22

<210> 3

<211> 20

<212> DNA

<213> Artificial Sequence

<220>

<223> primer

<400> 3
cttctactgc gattctcctg 20

<210> 4

<211> 20

<212> DNA

<213> Artificial Sequence

<220>

<223> primer

<400> 4
cttgccctcc ttgatgttat 20

<210> 5

<211> 20

<212> DNA

<213> Artificial Sequence

<220>

<223> primer

<400> 5
tggatgggat tgaggagaag 20

<210> 6

<211> 20

<212> DNA

<213> Artificial Sequence

<220>

<223> primer

<400>  6
aaaccacagt cctcacaggg                                                    20

<210>  7

<211>  18

<212>  DNA

<213>  Artificial Sequence


<220>

<223>  primer

<400>  7
ccaacctacc ccccaatc                                                      18

<210>  8

<211>  20

<212>  DNA

<213>  Artificial Sequence


<220>

<223>  primer

<400>  8
gcctcagagg cagtgttttc                                                    20

<210>  9

<211>  21

<212>  DNA

<213>  Artificial Sequence


<220>

<223>  primer

<400>  9
caggagcaag aggtcattct g                                                  21

<210>  10

<211>  22

<212>  DNA

<213>  Artificial Sequence

<220>

<223>  primer

<400>  10
tgttatcaaa gatggcttgg tg                                                22

<210>  11

<211>  21

<212>  DNA

<213>  Artificial Sequence


<220>

<223>  primer

<400>  11
gctgacctca gatgtgagag g                                                 21

<210>  12

<211>  22

<212>  DNA

<213>  Artificial Sequence


<220>

<223>  primer

<400>  12
tcccattgaa gatgtacaca gc                                                22

<210>  13

<211>  20

<212>  DNA

<213>  Artificial Sequence


<220>

<223>  primer

<400>  13
tacagagtga ggcggtttga                                                   20

<210>  14

<211>  20

<212>  DNA

<213>  Artificial Sequence

<220>
<223>  primer

<400>  14
agcgaagcct ggagagtatt                                                    20

<210>  15
<211>  20
<212>  DNA
<213>  Artificial Sequence

<220>
<223>  primer

<400>  15
cgagaagaag ggagaatcac                                                    20

<210>  16
<211>  20
<212>  DNA
<213>  Artificial Sequence

<220>
<223>  primer

<400>  16
cataaggcaa ccacaccatc                                                    20

<210>  17
<211>  20
<212>  DNA
<213>  Artificial Sequence

<220>
<223>  primer

<400>  17
agcccagagt atgttccaga                                                    20

<210>  18

<211>  20

<212>  DNA

<213>  Artificial Sequence

<220>

<223>  primer

<400>  18
ctcaaactca tcgctcatcc                                                    20

<210>  19

<211>  20

<212>  DNA

<213>  Artificial Sequence

<220>

<223>  primer

<400>  19
tgctacaggg tttcatccag                                                    20

<210>  20

<211>  20

<212>  DNA

<213>  Artificial Sequence

<220>

<223>  primer

<400>  20
caaagtagaa gagggcaacc                                                    20

<210>  21

<211>  25

<212>  DNA

<213>  Artificial Sequence

<220>

<223>  primer

<400>  21
tggaatcctg tggcatccat gaaac                                              25

<210>  22

<211>  25

<212>  DNA

<213>  Artificial Sequence

<220>

<223>  primer

<400>  22
taaaacgcag ctcagtaaca gtccg                                              25

<210>  23

<211>  208

<212>  PRT

<213>  Homo sapiens

<400>  23

Met Asp Pro Phe Leu Val Leu Leu His Ser Val Ser Ser Ser Leu Ser
1               5                   10                  15

Ser Ser Glu Leu Thr Glu Leu Lys Phe Leu Cys Leu Gly Arg Val Gly
            20                  25                  30

Lys Arg Lys Leu Glu Arg Val Gln Ser Gly Leu Asp Leu Phe Ser Met
        35                  40                  45

Leu Leu Glu Gln Asn Asp Leu Glu Pro Gly His Thr Glu Leu Leu Arg
    50                  55                  60

Glu Leu Leu Ala Ser Leu Arg Arg His Asp Leu Leu Arg Arg Val Asp
65                  70                  75                  80

Asp Phe Glu Ala Gly Ala Ala Ala Gly Ala Ala Pro Gly Glu Glu Asp
                85                  90                  95

Leu Cys Ala Ala Phe Asn Val Ile Cys Asp Asn Val Gly Lys Asp Trp
            100                 105                 110

Arg Arg Leu Ala Arg Gln Leu Lys Val Ser Asp Thr Lys Ile Asp Ser
        115                 120                 125

Ile Glu Asp Arg Tyr Pro Arg Asn Leu Thr Glu Arg Val Arg Glu Ser
    130                 135                 140

Leu Arg Ile Trp Lys Asn Thr Glu Lys Glu Asn Ala Thr Val Ala His
145                 150                 155                 160

Leu Val Gly Ala Leu Arg Ser Cys Gln Met Asn Leu Val Ala Asp Leu
                165                 170                 175

Val Gln Glu Val Gln Gln Ala Arg Asp Leu Gln Asn Arg Ser Gly Ala
            180                 185                 190

Met Ser Pro Met Ser Trp Asn Ser Asp Ala Ser Thr Ser Glu Ala Ser
        195                 200                 205

<210>  24

<211>  205

<212>  PRT

<213>  Mus musculus


<400>  24

Met Asp Pro Phe Leu Val Leu Leu His Ser Leu Ser Gly Ser Leu Ser
1               5                   10                  15

Gly Asn Asp Leu Met Glu Leu Lys Phe Leu Cys Arg Glu Arg Val Ser
            20                  25                  30

Lys Arg Lys Leu Glu Arg Val Gln Ser Gly Leu Asp Leu Phe Thr Val
        35                  40                  45

Leu Leu Glu Gln Asn Asp Leu Glu Arg Gly His Thr Gly Leu Leu Arg
    50                  55                  60

Glu Leu Leu Ala Ser Leu Arg Arg His Asp Leu Leu Gln Arg Leu Asp
65                  70                  75                  80

Asp Phe Glu Ala Gly Thr Ala Thr Ala Ala Pro Pro Gly Glu Ala Asp
                85                  90                  95

Leu Gln Val Ala Phe Asp Ile Val Cys Asp Asn Val Gly Arg Asp Trp
            100                 105                 110

Lys Arg Leu Ala Arg Glu Leu Lys Val Ser Glu Ala Lys Met Asp Gly
        115                 120                 125

Ile Glu Glu Lys Tyr Pro Arg Ser Leu Ser Glu Arg Val Arg Glu Ser
    130                 135                 140

Leu Lys Val Trp Lys Asn Ala Glu Lys Lys Asn Ala Ser Val Ala Gly
145                 150                 155                 160

Leu Val Lys Ala Leu Arg Thr Cys Arg Leu Asn Leu Val Ala Asp Leu
                165                 170                 175

Val Glu Glu Ala Gln Glu Ser Val Ser Lys Ser Glu Asn Met Ser Pro
            180                 185                 190

Val Leu Arg Asp Ser Thr Val Ser Ser Ser Glu Thr Pro
        195                 200                 205

<210>  25

<211>  360

<212>  PRT

<213>  Homo sapiens


<400>  25

Met Ser Gln Glu Arg Pro Thr Phe Tyr Arg Gln Glu Leu Asn Lys Thr
1               5                   10                  15

Ile Trp Glu Val Pro Glu Arg Tyr Gln Asn Leu Ser Pro Val Gly Ser
            20                  25                  30

Gly Ala Tyr Gly Ser Val Cys Ala Ala Phe Asp Thr Lys Thr Gly Leu
        35                  40                  45

Arg Val Ala Val Lys Lys Leu Ser Arg Pro Phe Gln Ser Ile Ile His
    50                  55                  60

Ala Lys Arg Thr Tyr Arg Glu Leu Arg Leu Leu Lys His Met Lys His
65                  70                  75                  80

Glu Asn Val Ile Gly Leu Leu Asp Val Phe Thr Pro Ala Arg Ser Leu
                85                  90                  95

Glu Glu Phe Asn Asp Val Tyr Leu Val Thr His Leu Met Gly Ala Asp
            100                 105                 110

Leu Asn Asn Ile Val Lys Cys Gln Lys Leu Thr Asp Asp His Val Gln
        115                 120                 125

Phe Leu Ile Tyr Gln Ile Leu Arg Gly Leu Lys Tyr Ile His Ser Ala
    130                 135                 140

Asp Ile Ile His Arg Asp Leu Lys Pro Ser Asn Leu Ala Val Asn Glu
145                 150                 155                 160

Asp Cys Glu Leu Lys Ile Leu Asp Phe Gly Leu Ala Arg His Thr Asp
                165                 170                 175

Asp Glu Met Thr Gly Tyr Val Ala Thr Arg Trp Tyr Arg Ala Pro Glu
            180                 185                 190

Ile Met Leu Asn Trp Met His Tyr Asn Gln Thr Val Asp Ile Trp Ser
        195                 200                 205

Val Gly Cys Ile Met Ala Glu Leu Leu Thr Gly Arg Thr Leu Phe Pro
    210                 215                 220

Gly Thr Asp His Ile Asp Gln Leu Lys Leu Ile Leu Arg Leu Val Gly
225                 230                 235                 240

Thr Pro Gly Ala Glu Leu Leu Lys Lys Ile Ser Ser Glu Ser Ala Arg
                245                 250                 255

Asn Tyr Ile Gln Ser Leu Thr Gln Met Pro Lys Met Asn Phe Ala Asn
            260                 265                 270

Val Phe Ile Gly Ala Asn Pro Leu Ala Val Asp Leu Leu Glu Lys Met
        275                 280                 285

Leu Val Leu Asp Ser Asp Lys Arg Ile Thr Ala Ala Gln Ala Leu Ala
    290                 295                 300

His Ala Tyr Phe Ala Gln Tyr His Asp Pro Asp Asp Glu Pro Val Ala
305                 310                 315                 320

Asp Pro Tyr Asp Gln Ser Phe Glu Ser Arg Asp Leu Leu Ile Asp Glu
                325                 330                 335

Trp Lys Ser Leu Thr Tyr Asp Glu Val Ile Ser Phe Val Pro Pro Pro
            340                 345                 350

Leu Asp Gln Glu Glu Met Glu Ser
        355                 360

<210>  26

<211>  360

<212>  PRT

<213>  Mus musculus


<400>  26

Met Ser Gln Glu Arg Pro Thr Phe Tyr Arg Gln Glu Leu Asn Lys Thr
1               5                   10                  15

Ile Trp Glu Val Pro Glu Arg Tyr Gln Asn Leu Ser Pro Val Gly Ser
            20                  25                  30

Gly Ala Tyr Gly Ser Val Cys Ala Ala Phe Asp Thr Lys Thr Gly His
        35                  40                  45

Arg Val Ala Val Lys Lys Leu Ser Arg Pro Phe Gln Ser Ile Ile His
    50                  55                  60

Ala Lys Arg Thr Tyr Arg Glu Leu Arg Leu Leu Lys His Met Lys His
65                  70                  75                  80

Glu Asn Val Ile Gly Leu Leu Asp Val Phe Thr Pro Ala Arg Ser Leu
                85                  90                  95

Glu Glu Phe Asn Asp Val Tyr Leu Val Thr His Leu Met Gly Ala Asp
            100                 105                 110

Leu Asn Asn Ile Val Lys Cys Gln Lys Leu Thr Asp Asp His Val Gln
        115                 120                 125

Phe Leu Ile Tyr Gln Ile Leu Arg Gly Leu Lys Tyr Ile His Ser Ala
    130                 135                 140

Asp Ile Ile His Arg Asp Leu Lys Pro Ser Asn Leu Ala Val Asn Glu
145                 150                 155                 160

Asp Cys Glu Leu Lys Ile Leu Asp Phe Gly Leu Ala Arg His Thr Asp
                165                 170                 175

Asp Glu Met Thr Gly Tyr Val Ala Thr Arg Trp Tyr Arg Ala Pro Glu
            180                 185                 190

Ile Met Leu Asn Trp Met His Tyr Asn Gln Thr Val Asp Ile Trp Ser
        195                 200                 205

Val Gly Cys Ile Met Ala Glu Leu Leu Thr Gly Arg Thr Leu Phe Pro
    210                 215                 220

Gly Thr Asp His Ile Asp Gln Leu Lys Leu Ile Leu Arg Leu Val Gly
225                 230                 235                 240

Thr Pro Gly Ala Glu Leu Leu Lys Lys Ile Ser Ser Glu Ser Ala Arg
                245                 250                 255

Asn Tyr Ile Gln Ser Leu Ala Gln Met Pro Lys Met Asn Phe Ala Asn
            260                 265                 270

Val Phe Ile Gly Ala Asn Pro Leu Ala Val Asp Leu Leu Glu Lys Met
        275                 280                 285

Leu Val Leu Asp Ser Asp Lys Arg Ile Thr Ala Ala Gln Ala Leu Ala
    290                 295                 300

His Ala Tyr Phe Ala Gln Tyr His Asp Pro Asp Asp Glu Pro Val Ala
305                 310                 315                 320

Asp Pro Tyr Asp Gln Ser Phe Glu Ser Arg Asp Leu Leu Ile Asp Glu
                325                 330                 335

Trp Lys Ser Leu Thr Tyr Asp Glu Val Ile Ser Phe Val Pro Pro Pro
            340                 345                 350

Leu Asp Gln Glu Glu Met Glu Ser
        355                 360

## Description

Apoptosis, or programmed cell death, is a process involved in development and homeostasis. Fas and Fas Ligand (FasL), members of the tumour necrosis factor (TNF) receptors/TNF families, are implicated in this process. Although Fas is constitutively expressed on most cells of the body, FasL has a more restrictive pattern of expression. FasL is expressed on activated T lymphocytes, where it contributes to cytotoxicity and to the regulation of the immune response by activation-induced cell death (AICD). FasL is also expressed in Sertoli cells of the testis, and in cells of the anterior chamber of the eye, where it contributes to the immune privilege status of these sites by killing infiltrating lymphocytes that express the receptor Fas. Recently, a new concept has emerged showing that Fas/FasL interactions may not only induce apoptosis of invading cytotoxic lymphocytes, but may also stimulate their own proliferation through Fas/FasL interactions.

Dysregulation of the Fas pathway is responsible of hypo- or hyperproliferative disorders that are due to excess or default of apoptosis, respectively. Some reports suggest that FasL expression on tumour cells enables these cells to escape the immune system (counter-attack model), although this point is still controversial. Usually, when the tumour cells co-express Fas and FasL molecules, they turn out to be resistant to Fas-mediated cell death. Among the regulatory mechanisms that may take place to block the death signal, down-regulation of pro-apoptotic molecules like Fas or caspase 8, or up-regulation of anti-apoptotic molecule like FLIP (FLICE inhibitory protein) have been reported during tumour progression.

In susceptible cells, activation of Fas by its ligand leads to the subsequent recruitment of the adapter molecule FADD (Fas-associated protein with death domain) which in turn activates FLICE/caspase 8, a process that leads to apoptosis. FLIP, an anti-apoptotic protein, can protect cells from programmed cell death by preventing association of caspase 8 with the adapter molecule FADD.

Daxx (Fas death domain-associated protein), a second adapter molecule for the Fas receptor, defines a different signalling pathway downstream of Fas. It can activate independently the Jun N-terminal kinases (JNKs) or p38 mitogen-activated protein kinases (p38 MAPKs) pathways through the ASK1 (apoptosis signal-regulating kinase 1) intermediate. Whether Daxx signalling leads to induction of death or proliferation is not clear. It seems that JNK pathway generally leads to apoptosis whereas p38 kinase have been described as having pro- or anti-apoptotic effects. The final result therefore appears to depend from multiple factors such as the type of cells and its state of differentiation.
The kinase RIP (receptor-interacting protein), was recently shown to be a third adapter for Fas that can transduce Fas-mediated caspase-dependent or - independent cell death, further increasing the complexity of the Fas signalling pathway.

Faced with the still increasing complexity of the Fas/FasL pathway transducing machinery, the inventors first made the choice of a particular model in which Fas/FasL interactions have a particularly intriguing role, namely the thyroid organ. Indeed, in thyroid follicular cells (TFC), the role of the Fas pathway is highly debated, and has been the focus of multiple studies. The overall idea is that normal thyrocytes constitutively express the Fas molecule, and that FasL expression can occur under numerous thyroid pathological conditions including Hashimoto's thyroiditis (HT), Grave's disease and cancer. Whether thyrocyte FasL expression is beneficial or detrimental is more confusing and certainly depends from the type of thyroid disease. For example, in Hashimoto's thyroiditis (HT), Fas-FasL interactions among HT thyrocytes could lead to suicide/fratricide of the cells, and consequent hypothyroidism. By contrast, FasL expression by TFC in cancer and Grave's disease has been postulated to help the thyroid cells to escape immune system. One observation which has held the inventors' attention is that TFC are resistant to Fas-mediated cell death under normal conditions, and that some reports indicated that massive Fas-mediated apoptosis of TFC could be obtained after treatment with IL-1beta or IFNgamma, whereas other reports indicated that they were unable to induce cell death under the same conditions, but could succeed with a combination of IL-1beta and IFNgamma, while some still other reports indicated that IL-1beta and/or IFNgamma do not induce TFC apoptosis, but rather a blockage of proliferation. Hence, due to the controversy arising from non confluent experimental results, it has been difficult to address experimentally the issue of Fas pathway regulation in general, and of Fas pathway regulation in the thyroid organ in particular.

Faced with these technical problems, the inventors then made the further choice to direct their focus towards in *vivo* models, namely non-human mammals which have been genetically engineered so as to develop thyroid tumours. They notably made the choice of using transgenic mice which develop tumour through a constitutive activation of adenylate cyclase. These mice have no abnormality up to 8 months of age, and thereafter develop hyperplasia followed by hyperfunctioning thyroid adenomas, or adenocarcinomas depending of the genetic background of the mice. Such a constitutive activation of adenylate cyclase is observed in approximately 30% of human thyroid toxic adenomas and 10% of human thyroid carcinomas. The inventors thereby selected a non-human model which has the particular feature of being a powerful model for studying human tumours.

The inventors then further chose to investigate a large array of those signalling molecules involved in the Fas cascade in gsp transgenic mice, and to combine three different techniques (semi-quantitative RT-PCR, western blot and immunohistochemistry) to detect and quantify molecules at both mRNA and protein amounts, and to localize protein expressing cells.

The results thus obtained show that, under *in vivo* conditions, normal thyroid follicular cells (TFC) of non transgenic (NTG) mice, like non pathological gland of transgenic mice, do not express FasL molecule. By contrast, FasL expression is acquired during *in vivo* tumoural development with a higher expression in adenoma/adenocarcinoma than in hyperplastic thyroids. This up-regulation of FasL on tumour cells is correlated with a down-regulation of molecules of the Fas signalling pathway.

Among the three known Fas receptor adapters (FADD, Daxx, RIP), only FADD was highly regulated. Moreover, this FADD regulation during tumour development has a very striking feature: indeed, the inventors found that although FADD mRNA was present, FADD protein expression was very weak or completely lost in tumour.
The inventors have furthermore observed that if *ex vivo* normal thyroid cells express both FADD mRNA and protein, they however loose FADD expression when placed under standard *in vitro* TFC culture conditions. They have also observed that some tumour cell lines, such as for example the K562 cell line, express FADD, whereas the corresponding *ex vivo* tumour, such as CML (chronic myeloid leukaemia) in the case the K652 cell line, does in fact not express FADD. Those prior art results which were based on *in vitro* cultures, or on established cell lines cannot therefore be directly transposed to the actual *in vivo* conditions. Apart from evidencing that such prior art experiments have suffered from important artefacts, the inventors thereby demonstrate that, in the absence of FADD, Fas-signalling results in faster cell growth (and not in apoptosis).
The inventors also demonstrate that during *in vivo* tumour development, Fas signalling, which is deprived of FADD, acts mainly through a Daxx-ASKI pathway, ASK1 expression being strongly increased during tumour growth. As to the downstream kinases of this Daxx-ASK1 pathway, the inventors demonstrate that the ASK1/SEK1/JNK pathway, as well as the ASK1/MKK3/p38-MAPK pathway are strongly inhibited during *in vivo* tumour development. More particularly, they show that during *in vivo* tumour development:
- there is a decrease of total MKK3 expression,
- among MKK3/MKK6 proteins, only MKK6 proteins are activated,
- the activation of p38-MAPK is inhibited (decrease of phospho-p38-MAPK), and that this inhibition correlates with the decrease in total MKK3 expression,
- the expression amount of total SEK1 proteins strongly increases, but the amount of the thr261 phospho-active form of SEK1 strongly decreases, and that
- SAPK/JNK proteins are phosphorylated at both thr183 and thr185 sites in non pathological organs, but that hyperplastic organs and tumours are mono-phosphorylated due to a lack of phospho-SEK1 dependent thr183 phosphorylation.
   The inventors have thus demonstrated that during the transformation process from non pathological organ to tumour:
   1) FasL expression is gained,
   2) FADD protein but not FADD mRNA is lost,
   3) Daxx and RIP amounts are not significantly affected,
   4) Fas signalling in the absence of FADD results in a Daxx- - but not RIP- mediated signal,
   5) there is an increase in ASK1 expression amount (Daxx-ASK1 pathway),
   6) disappearance of FADD protein is correlated with an inactivated status of p38-MAPK (inhibition of p38-MAPK phosphorylation),
   7) there is a switch from dual to mono-phosphorylation of SAPK/JNK proteins
   8) there is an increase in total SEK1 protein expression amount, and with a decrease in the amount of thr261 phospho-active form of SEK1,
   9) Fas signalling in absence of FADD promotes cell growth instead of apoptosis.

This is the first instance where spontaneous FADD protein disappearance has been associated with actual *in vivo* pathological consequences, and that a decrease in phosphorylated p38-MAPK proteins has been determined as closely correlated with this decrease in FADD proteins.

Indeed, prior art results on FADD solely concerned models which had been engineered by man to have a deficient FADD function. Models based on gene targeting, or on expression of dominant negative interfering mutant have thus been described on T lymphocytes, and were shown to render T cells resistant to apoptosis induced by Fas (Newton *et al.* 1998, The EMBO Journal 17(3):706-718). In these models, engineered FADD defects also resulted in an inhibition of mitogen-induced T-cell proliferation, demonstrating that FADD may transmit both apoptotic and growth and/or survival signals. Conversely, another model demonstrated that an engineered FADD deficiency in rag-1^{-/-} mice induced the development of thymic lymphoma, indicating that FADD may also act as a lymphoma suppressor (Newton *et al.*, 2000, The EMBO Journal Vol. 19 No.5 pp. 931-941). These prior art models mainly demonstrated that FADD protein might have various effects depending on the time of its expression, the tissue and the development status of the tissue.

In the present *in vivo* tumour model, the inventors demonstrate that there is a spontaneous loss of FADD expression, and more particularly that FADD undergoes post-translational regulations during the development of the tumour. This is the first description that the decrease in FADD proteins is a reliable marker of tumour progression, and there is a closely correlated decrease in phosphorylated p38-MAPK.

None of the prior art models, in which FADD function was deliberately altered by man, so as to elucidate the mechanisms by which Fas is regulated, does suggest that there is an actual loss in FADD expression during *in vivo* tumoural progression, that this loss affects FADD protein but not FADD mRNA, and that there may be a correlated decrease in phosphorylated p38-MAPK. Of particular note in this respect is that, according to the present invention, there is no significant regulation of Daxx and RIP, whereas Daxx and RIP have been described in prior art models as apoptosis potentiators (see *e.g.* US 5,674,734 Leder *et al.* ; US 6,159,731 Yang *et al*.). This illustrates that one cannot deduce from models which have been engineered to be deficient in a particular molecule, and which thereby show that this particular molecule favours (or, on the contrary, prevents from) a tumour development, that this particular molecule actually is a biological marker of tumour development (or regression, respectively).

The results obtained by the inventors on a non-human mammal model of adenoma/adenocarcinoma have further been confirmed on human patients, and on other types of tumours, such as notably acute and chronic myeloid leukaemia (see example 2 below): the decrease in FADD protein expression measured in a blood sample collected from a myeloid leukaemia patient reliably correlates with the aggressiveness of the disease. These results hence altogether fully confirm that the decrease in FADD proteins and/or in phosphorylated p38-MAPK is a reliable marker for the *in vivo* status of tumours such as adenoma, adenocarcinoma, myeloid leukaemia, and that it is applicable to human persons as well as to non-human animals.

Faced with the stricking demonstration that there is a FADD protein but not a FADD mRNA cell loss during tumour development, the inventors have further on focussed on how such a post-translational regulation may operate.
Upon testing of different candidate mechanisms, the inventors have come to the demonstration that FADD protein is in fact secreted or excreted from out of the tumour-transforming cells to the extracellular environment. They notably demonstrate that this secretion/excretion mechanism actually happens in human cancer patients, wherein FADD protein is lost from tumour cells and detected in the serum. They also demonstrate that, in its secreted/excreted form, FADD protein is detected at a MW around 30-33 kDa whereas in its cellularly-expressed form it has a MW of about 28-29 kDA, and that the 30-33 kDa extracellular form comes down to a 28-29 kDa form upon treatment with a reducing agent such as 2-mercaptoethanol, thus suggesting that the extracellularly secreted/excreted FADD protein is linked by disulfide bonds or bridages to another entity.

Coming deeper in the medical applications deriving from these surprising new features, the inventors also demonstrate *via* clinical analysis of the development of tumours such as adenomas, adenocarcinomas, myeloid tumours (and notably, myeloid leukaemias, such as CML and AML), that, in parallel to what is observed with the amount of cellular FADD proteins, it is observed that the amount of extracellular FADD proteins is correlated with the tumour status (in a mirror-reversed fashion compared to cellular FADD proteins): a high or above-normal amount of extracellular FADD proteins is correlated with tumour presence, development or high aggressiveness, and a null, low or sub-normal amount of extracellular FADD proteins is correlated with tumour absence, regression or low aggressiveness.

The inventors further demonstrate that the amount of cellular FADD proteins and/or the extracellular amount of FADD proteins can be predictive of resistance to chemotherapy. They notably demonstrate that there is a (statistically significant) correlation between low amount of cellular FADD proteins or high amount of extracellular FADD proteins, and resistance to chemotherapy. They notably demonstrate that:
- a null, low or sub-normal level of cellular expression of FADD protein is correlated with resistance to anti-myeloid tumour chemotherapy, and that
- a high or above-normal level of extracellular presence of FADD protein is correlated with resistance to anti-myeloid tumour chemotherapy.

It should however be noted that a null, low or sub-normal level of extracellular presence of FADD protein is not always a conclusive data by itself: in that case, it is therefore recommended to assess the level of FADD cellular expression, and if this FADD cellular level is assessed as null, low or sub-normal, then conclusion of resistance can be reliably reached.

The invention hence also relates to the use of a FADD protein for the prognosis of resistance to an anti-tumour (and/or anti-mitotic) chemotherapy, and notably for the prognosis of resistance to an anti-myeloid tumour chemotherapy, such as a chemotherapy against a CML (Chronic Myeloid Leukaemia) or an AML (Acute Myeloid Leukaemia).

In other words, the inventors have demonstrated that:
- the disappearance of cellular FADD proteins and the correlated disappearance of cellular phosphorylated p38-MAPKs are reliable cell markers for the *in vivo* presence or development of a tumour, and the appearance of extracellular FADD proteins is a reliable extracellular marker for the *in vivo* presence or development of a tumour, and, conversely,
- the non-disappearance or the re-appearance of cellular FADD proteins and of cellular phosphorylated p38-MAPKs are reliable cell markers for the *in vivo* absence or regression of a tumour, and the non-appearance or disappearance of extracellular FADD proteins is a reliable extracellular marker for the *in vivo* presence or development of a tumour.
   They notably demonstrate that these relations are particularly reliable for adenomas, adenocarcinomas, and more particularly for thyroid adenomas or adenocarcinomas.
   They also demonstrate that FADD proteins are reliable cell and extracellular markers for determining the *in vivo* development of a special type of tumour, namely the *in vivo* development of a tumour which has the capacity to resist to standard anti-tumour chemotherapy. They demonstrate that this relation with chemoresistance status is particularly reliable for myeloid tumours, such as CML or AML.

The invention provides hence generally provides with methods and kits that are helpful for diagnosing the presence of a tumour in an animal, for determining the *in vivo* efficiency of a tentative anti-tumour treatment, and for determining the danger rating a compound may have *in vivo* in terms of pro-tumour activity.
The inventors notably provide with diagnosis, prognosis, and therapeutic applications which are notably based on this FADD secretion/excretion mechanism, and notably with easy-to-handle diagnosis means that can advantageously be performed on extracellular samples of tumours, with prognosis means which allow to avoid treating patients with chemotherapy that in fact cannot succeed (thereby also allowing to avoid undesired resistance from developing against the chemotherapeutic agent), and with therapeutically-useful means which makes use of the anti-tumour property the function of preventing FADD from being extracellularly released.

The present patent application thus generally relates to the use of a protein selected from the group consisting of FADD proteins and phosphorylated p38-MAPK, as a tumour indicator. Indeed:
- a null, low or sub-normal amount of cellular FADD proteins is correlated with tumour presence or development, and a high or above-normal amount of extracellular FADD proteins is also correlated with tumour presence or development, and
- a high or above-normal amount of cellular FADD proteins is correlated with tumour absence or regression, and a null, low or sub-normal amount of extracellular FADD proteins is also correlated with tumour absence or regression.

These relationships are particularly reliable for adenomas and adenocarcinomas, and more particularly for thyroid adenomas and adenocarcinomas. When said tumour is or is suspected to be in an animal, such as a mammal and notably a human patient, said extracellular environment can e.g. be serum or plasma.
As above-indicated, and as further illustrated in the below examples, the level of FADD expression as well as of the amount of phosphorylated p38-MAPK are indeed most generally high in non-tumour animal tissue or fluid, and drastically decrease, sometimes down to zero, when a tumour develops from these tissue or fluid. As a parallel feature, expression of FasL is gained.
In addition to the detection of a decrease in cellular FADD proteins and/or phosphorylated p38-MAPK, it is according to the invention advantageously further made use of FasL protein as a cell marker of which gained expression indicates that a tumour is present, or in development, or in spreading in an animal. It has however to be noted that the adapter FADD is not exclusively involved in the Fas pathway: FADD is also involved in the pathways of a wide variety of other death receptors, such as TNFR1, DR3, DR4 and DR5.

By "amount" or "content" of a protein, it is herein meant a quantitative amount such as quantity, concentration, level of presence, level of expression, but it is also meant to encompass a qualitative assessment such as presence/absence (an amount not significantly different from zero means absence ; absence represents an amount that is inferior to presence, and presence represents an amount that is superior to absence).

Such an amount may be measured by any technique that is convenient for the qualitative or quantitative assessment of proteins, such as ELISA or Western blots techniques. Such an amount then be expressed in units such as concentration, OD value, band intensity.
The term "significant", when used in the comparison of two protein amounts, means that the result of this comparison is statistically significant with the technique that has been used to measure said amounts.
Any statiscal test that is found appropriate by the skilled person may be used, *e.g.* a Fisher test, Student test.

The present invention hence provides with a method to determine a status of tumour absence/presence or regression/development, characterized in that said status is determined:
- by evaluating whether the cells of said tumour or suspected tumour have, when compared to non-tumour but otherwise equivalent cells, lost an abnormal amount of FADD proteins, and/or when they have extracellularly released an abnormal amount of FADD proteins, and/or
- by evaluating whether the cells of said tumour or suspected tumour have, when compared to non-tumour but otherwise equivalent cells, an abnormally low amount of phosphorylated p38-MAPKs.

The present invention thus encompasses a method and a kit to determine whether cells have or not turned into tumour cells in an organism such as an animal, characterized in that:
at least one amount selected from the group consisting of the amount of FADD proteins and the amount of phosphorylated p38-MAPK is measured in said cells, and
wherein said cells are determined as tumour cells, when said measured amount(s) either is(are) not significantly different from zero, and/or is(are) significantly inferior to the standard normal (respective) amount(s) which is(are) observed in non-tumour but otherwise equivalent cells, and
wherein said cells are determined as non-tumour cells, when said measured amount(s) is(are) significantly different from zero, and either not significantly different from, or significantly superior to said standard (respective) normal amount(s).
This method is particularly reliable for adenomas and adenocarcinomas, and more particularly for thyroid adenomas and adenocarcinomas.

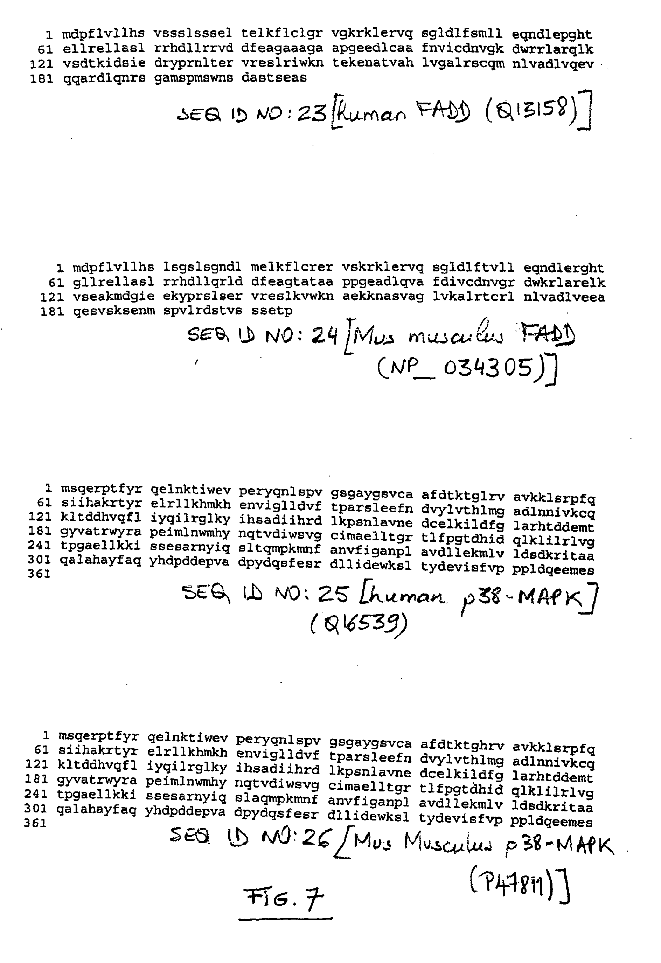

FADD proteins have an apparent MW of 28-30 kDa on Western blot, and have the amino acid sequence SEQ ID NO:23 in humans (accession Q13158), SEQ ID NO:24 in mice (accession NP_034305). When it is in its extracellularly released form (e.g. in the serum or plasma of an animal), FADD proteins are detected at a MW of about 30-33 kDa on Western blot, and are then most likely linked to another or other entities by disulfide bonds or bridges.
Phosphorylated p38-MAPK have an apparent MW of 42-44 kDa on western blot, and p38-MAPK has the amino acid sequence SEQ ID NO:25 in humans (accession Q16539), SEQ ID NO:26 in mice (accession P47811). SEQ ID NO:23-26 are also shown on Figure 7.

FADD as well as phosphorylated p38-MAPK have an ubiquitous panel of expression under physiological conditions, which implies that the vast majority of cells collected from an animal tissue or fluid should express FADD proteins and contain phosphorylated p38-MAPK when they have a non-tumour status, and should have a decreased amount of, or an absence of FADD proteins and of phosphorylated p38-MAPK, when they develop a tumour status. The method of the invention is therefore pertinent to the vast majority of cells such as animal cells, except those cells which would, by exception, not express FADD under tumour-free normal conditions, and would not have phosphorylated p38-MAPK under activated but tumour-free normal conditions. It is at this stage reminded that, as above explained and below illustrated, it is of first importance for the present invention, to consider those FADD and/or phospho-p38-MAPK amounts which are actually present *in vivo,* for example by analysing an *ex vivo* sample, and to not directly rely on measures performed on *in vitro* cultures or on established cell lines as these later experimental conditions may introduce artefacts in this respect (this is notably the case for *in vitro* standard cultures of non-tumour thyrocytes).

The skilled person is therefore invited to proceed to the usual control analysis which is the standard procedure in biotechnology, so as to check that when they do not have a tumour status, the tested tissue, fluid or cells actually express FADD proteins and/or phosphorylated p38-MAPK *in vivo*, thereby measuring those amounts which are hereinafter referred to as the standard (respective) normal amounts. Under non-tumour conditions, FADD should indeed be expressed, and p38-MAPK should be phosphorylated when the cell is activated.

The present application hence generally relates to the use of at least one protein selected from the group consisting of FADD proteins and phosphorylated p38-MAPKs, as a indicator of tumour status. By "tumour status", it is herein meant any medically-relevant property of a tumour, such as its growth or growth potential. This term therefore encompasses a status of presence/absence or development/regression, as well as a status of chemoresistance/chemosensitivity.
FADD proteins and phosphorylated p38-MAPKs can constitute cell markers for absence of tumour. Indeed, the cellular amount of such a protein can be a cell indicator of presence/absence or development/regression of a tumour. Similarly, the extracellular amount of FADD proteins can be an extracellular indicator of presence/absence or development/regression of a tumour.
Said tumour is preferably an adenoma, or an adenocarcinoma, more preferably it is a thyroid adenoma or adenocarcinoma. Advantageously, said tumour is an *in vivo* tumour.
This use may further be complemented by the step of using a FasL protein as a cell marker for presence of *in vivo* tumour. The (respective) cellular amount(s) of FADD proteins and phosphorylated p38-MAPK indeed decrease sometimes up to zero with tumour development, while FasL expression is gained. All the methods and uses of the invention may therefore advantageously comprise the detection of the presence or absence, or the measure of the cellular amount of FasL proteins. Such a detection or measure can be achieved by anti-FasL specific compounds, such as anti-FasL specific antibodies (*e.g.* the anti-FasL IgG2A H11 manufactured by Alexis Biochemicals ; the rabbit polyclonal anti-FasL antibodies Q20 and C20 available from Santa Cruz Biotechnology ref.sc-956 and ref.sc-957, respectively ; the rabbit polyclonal anti-FasL (Ab-3) available from Oncogene Research ref. PC105 ; the mouse monoclonal anti-FasL (G247-4) available from BD PharMingen ref.65431A; the mouse monoclonal anti-FasL (NOK-1)BD PharMingen ref. 65321A ; the mouse monoclonal anti-FasL (NOK-2) available from BD PharMingen ref. 65331A).
Such *in vivo* tumours notably include benign tumours such as adenomas, as well as cancers such as adenocarcinoma. The absence of expression of, or an abnormally low cellular amount of FADD proteins and/or phosphorylated p38-MAPK is a particularly reliable marker for thyroid toxic adenomas, thyroid carcinomas. These markers can be used for detecting a tumour in an organism, and notably in an animal such as a mammal, and advantageously in a human person.

According to a first aspect of the invention, the application relates to a method for determining whether a suspected tumour has developed or spread or is in development or spreading, or whether such a tumour is absent or has regressed near to remission or recovery. This method is characterised in that it comprises the following steps:
- measuring at least one cellular amount selected from the group consisting of the cellular amount of FADD proteins which is present in cells representative of said suspected tumour, and the cellular amount of phosphorylated p38-MAPKs which is present in cells representative of said suspected tumour,
wherein said tumour is determined as having developed or spread or as being in development or in spreading, when said measured cellular amount(s) either is(are) not significantly different from zero, and/or is(are) significantly inferior to the (respective) standard normal cellular amount(s) which is(are) observed in non-tumour but otherwise equivalent cells, and
wherein said tumour is determined as absent or as in regression near to remission or recovery, when said measured cellular amount(s) is(are) significantly different from zero and either not significantly inferior to said (respective) standard normal cellular amount(s), and/or, the following step:
- measuring the extracellular amount of FADD proteins which is present in the extracellular environment of cells that are representative of said suspected tumour,
wherein said tumour is determined as having developed or spread or as being in development or in spreading, when said measured extracellular amount of FADD proteins is significantly superior to the standard normal extracellular amount of FADD proteins which is observed in the extracellular environment of non-tumour but otherwise equivalent cells,
wherein said tumour is determined as absent or as in regression near to remission or recovery, when said measured extracellular amount of FADD proteins is either not significantly different from zero and/or not significantly superior to said standard normal extracellular amount of FADD proteins,
provided that the standard normal cellular amount(s) of FADD proteins and/or of phosphorylated p38-MAPKs, respectively, which is(are) observed in non-tumour but otherwise equivalent cells, is(are) significantly different from zero.
This method is particularly appropriate for adenomas and adenocarcinomas, and more particularly for thyroid adenomas and adenocarcinomas.

To measure a cellular amount of protein, a cell sample that is representative of said tumour may be provided by collection from the organism for whom there is said suspicion of tumour, or from a biological representative thereof (*e.g.* tissue samples for a suspicion of adenoma or adenocarcinoma, etc.).
To measure an extracellular amount of protein, an extracellular sample that is representative of the extracellular environment of cells which are representative of said suspected tumour may be provided by collection from the organism for whom there is said suspicion of tumour, or from a biological representative thereof. When said suspected tumour is an adenoma or adenocarcinoma, said extracellular environment can e.g. be the serum or the plasma.

To implement this method, it is highly recommended to measure the standard (respective) normal amount(s) which should be found in the particular sample being tested if it would not contain a tumour, so as to check that this(these) standard (respective) normal amount(s) actually is(are) significantly different from zero (thereby confirming that the FADD and phosphorylated p38-MAPK markers are applicable to the particular case under analysis). Preferably, said standard (respective) normal amount(s) corresponds(correspond) to the (respective) mean value(s) of a plurality of normal amounts, preferably of at least 5 normal amounts, more preferably of at least 10 normal amounts.
Such standard (respective) normal amount(s) are also helpful in that they represent precise reference values with which the measured ones can be accurately compared (thereby enabling an accurate diagnosis). One should however note that such an accuracy is not always necessary: for example, a total absence in FADD protein and/or phosphorylated p38-MAPK is by itself sufficient to conclude that a tumour is present or in development, if it has been checked that these proteins are present under non-tumour normal *in vivo* conditions.

More particularly, the application relates to a method for determining whether a tumour is in a phase of development or of spreading, whether it is in a phase of stabilization, or whether it is in a phase of regression, remission or recovery, which is characterised in that it comprises the following steps:
a. measuring at least one cellular amount selected from the group consisting of the cellular amount of FADD proteins which is present in cells representative of said tumour, and the cellular amount of phosphorylated p38-MAPKs which is present in cells representative of said tumour, this(these) measured cellular amount(s) being referred to as the measured first cellular amount(s),
b. reiterating step a. but at a later time, this(these) measured amount(s) being then referred to as the measured second cellular amount(s), and
   wherein said tumour is determined as being in a phase of development or of spreading, when said measured second cellular amount(s) is(are) significantly inferior to said measured (respective) first cellular amount(s),
   wherein said tumour is determined as being in a phase of stabilization, when said measured second cellular amount(s) is(are) not significantly different from said measured (respective) first cellular amount(s),
   wherein said tumour is determined as being in a phase of regression, remission or recovery, when said measured second cellular amount(s) is(are) significantly superior to said measured (respective) first cellular amount(s),
   and/or, the following steps:
c. measuring the amount of FADD proteins which is present in the extracellular environment of cells representative of said tumour, this measured amount being referred to as the measured first extracellular FADD amount,
d. reiterating step c. but at a later time, this measured amount being then referred to as the measured second extracellular FADD amount, and
wherein said tumour is determined as being in a phase of development or of spreading, when said measured second extracellular FADD amount is significantly superior to said measured first extracellular FADD amount,
wherein said tumour is determined as being in a phase of stabilization, when said measured second extracellular amount is not significantly different from said measured first extracellular amount,
wherein said tumour is determined as being in a phase of regression, remission or recovery, when said measured second extracellular amount is significantly inferior to said measured first extracellular amount,
provided that the standard normal cellular amount(s) of FADD proteins and/or of phosphorylated p38-MAPKs, respectively, which is(are) observed in non-tumour but otherwise equivalent cells, is(are) significantly different from zero.
This method is particularly appropriate for adenomas and adenocarcinomas, and more particularly for thyroid adenomas and adenocarcinomas.

Said tumour may be subjected to a tentative anti-tumour therapy after step a. but before step b., and/or after step c. but before step d.

The application also relates to a kit for determining absence/presence and/or regression/development of a tumour, such as adenomas, adenocarcinomas, and more particularly thyroid adenomas, thyroid adenocarcinomas, said kit comprising:
- at least one compound which is capable of specifically binding to FADD proteins and/or to a phosphorylated p38-MAPKs, and/or at least one biological unit which is capable of producing such a compound, this at least one compound or biological unit being optionally contained in a physiological buffer, and/or optionally bound to a solid support, and
- appropriate instructions:
   ○ for using a FADD protein and/or phosphorylated p38-MAPK as cell indicator(s) the cellular presence/absence of which and/or the level of cellular expression of which is(are) correlated with absence/presence and/or regression/development status of said tumour, and/or
   ○ for using a FADD protein as an extracellular indicator the extracellular absence/presence of which and/or the level of extracellular presence of which is(are) correlated with tumour absence/presence and/or regression/development.

More particularly, the present application relates to the use of a FADD protein for the prognosis of resistance to an anti-tumour chemotherapy. The amount of FADD proteins in cells which are representative of said tumour and/or in the extracellular environment of such cells is used as predictive of resistance to said anti-tumour chemotherapy. Preferably, said chemotherapy is anti-myeloid tumour chemotherapy. Examples of anti-tumour chemotherapy include an interferon treatment such as an alpha interferon treatment, a standard induction chemotherapy, an anthracycline treatment such as doxorubicin, a standard or high dose cytarabin treatment and a vepeside treatment, cytostatic used alone or in combination

The present invention hence provides with an *in* vitro-based prognosis of resistance to anti-tumour chemotherapy which is predictive of resistance of a human patient to an *in* vivo-intended anti-myeloid tumour chemotherapy.
The application thus relates to a method for the prognosis of a resistance of an organism to an anti-tumour chemotherapy, without having to implement said chemotherapy, characterized in that it comprises determining that FADD proteins have been released from cells which are representative of the myeloid tumour to which said chemotherapy is intended, in an amount which is highly superior to that of non-tumour but otherwise equivalent cells. For this chemo-resistance prognosis, said tumour preferably is a myeloid tumour, such as a CML or an AML.
According to the present invention, a method for the prognosis of the resistance of a tumour to an anti-tumour chemotherapy can therefore be performed without having to implement said chemotherapy. It comprises the following step:
- measuring the cellular amount of FADD proteins which is present in cells that are representative of the cells to which said anti-tumour chemotherapy is intended,
wherein said tumour is determined as resistant to said chemotherapy when said measured FADD cellular amount is significantly inferior to the cellular amount of FADD proteins which is present in the cells of an immortalized FADD-expressing myeloid or lymphoid cell line, such as the THP1 cell line (ATCC TIB202) or the U937 cell line (ATCC CRL1593), preferably when said measured FADD cellular amount is inferior to, preferably inferior by a factor of 1.5 to, more preferably by a factor of 1.7 to, very preferably by a factor of 1.8 to, most preferably twice as less the cellular amount of FADD proteins in such an immortalized FADD-expressing myeloid or lymphoid cell line,
and/or the following step:
- measuring the amount of FADD proteins which is present in the extracellular environment of the cells to which said anti-myeloid tumour chemotherapy is intended,
wherein said tumour is determined as resistant to said chemotherapy when said measured extracellular FADD amount is superior by a factor of at least two to, preferably by a factor of at least 2.5 to the standard normal extracellular amount of FADD proteins which is observed in the extracellular environment of non-tumour but otherwise equivalent cells,
provided that the standard normal cellular amount of FADD proteins which is observed in non-tumour but otherwise equivalent cells is significantly different from zero.

The THP1 cell line are the U937 cell line are both available from ATCC (Accession numbers TIB202 and CRL1593, respectively). They can be grown *in vitro* under standard culture conditions, such as at 37°C and CO₂ 5% on standard complete RPMI medium (RPMI 1640 Life Technologies, supplemented with 5% foetal bovine serum and with 100U/ml penicillin, 100µg/ml streptomycin), as described in the examples below.
Preferably, said myeloid tumour is a CML or an AML. The extracellular environment can e.g. be the serum or plasma of an animal.

The application also relates to a kit for the prognosis of resistance to an anti-tumour chemotherapy, and particularly to an anti-myeloid chemotherapy such as an anti-CML or anti-AML chemotherapy, characterized in that it comprises:
- at least one compound which is capable of specifically binding to FADD proteins, and/or at least one biological unit which is capable of producing such a compound, this at least one compound or biological unit being optionally contained in a physiological buffer, and/or optionally bound to a solid support, and
- appropriate instructions for using a FADD protein:
   ○ as a cell indicator the cellular absence of which and/or the level of cellular expression of which is(are) correlated with resistance to said anti-myeloid tumour chemotherapy, and/or
   ○ for using FADD proteins as an extracellular indicator the extracellular presence of which and/or the level of extracellular presence of which is(are) correlated with resistance to said anti-myeloid tumour chemotherapy.
   Optionally, the kit the prognosis of resistance according to the invention can also comprises an immortalized FADD-expressing myeloid or lymphoid cell line, such as the THP1 cell line or the U937 cell line.

Based on the method to determine tumour presence/absence or development/regression, and on the method to determine tumour high/low aggressiveness, the invention also provides with a method for determining whether a treatment is, or will be, a highly efficient anti-tumour treatment.

This method determines whether an applied treatment is, or will be, efficient to treat or to arrest the growth of a tumour which is present or in development or in spreading in an animal, and is characterised in that it comprises the following step:
- measuring at least one cellular amount selected from the group consisting of the cellular amount of FADD proteins which is present in cells representative of said tumour during the course or at the end of said treatment, and the cellular amount of phosphorylated p38-MAPKs which is present in cells representative of said tumour during the course or at the end of said treatment,
wherein said treatment is determined as efficient against said tumour, when said measured cellular amount(s) is(are) not significantly inferior to the (respective) standard normal cellular amount(s) which is(are) observed in non-tumour but otherwise equivalent cells,
and/or the following step:
- measuring the extracellular amount of FADD proteins which is present in the extracellular environment of cells representative of said tumour during the course or at the end of said treatment,
wherein said treatment is determined as efficient against said tumour, when said measured extracellular amount is either not significantly different from zero and/or not significantly superior to the standard normal extracellular amount of FADD proteins which is observed in non-tumour but otherwise equivalent cells,
provided that the standard normal cellular amount(s) of FADD proteins and/or of phosphorylated p38-MAPKs, respectively, which is(are) observed in non-tumour but otherwise equivalent cells, is(are) significantly different from zero.

To measure said cellular or extracellular amount, there can be provided a biological (cellular or extracellular) sample which originates from the organism
wherein there is said tumour, or from a representative biological model thereof (wherein a comparable tumour is present or in development or in spreading), during the course or at the end of said treatment, as a representative sample of said tumour. When the tumour is or develops in an animal, such as a mammal and notably a human person, said extracellular environment may be serum or plasma.

This method can be performed on the organism itself (*e.g.* an animal), but is of course preferably performed on a representative model in which a comparable tumour is present or in development or in spreading, when such a model is available. Such a representative model is notably useful when the organism for which the treatment efficiency has to be determined is a human patient. Such a model may then be a non-human mammal wherein a comparable tumour is present or developing (*e.g.* a mouse, a rabbit, a dog or a monkey which has been genetically engineered to express the same type of tumour as is expressed in said human patient, for example the TRAMP mouse model for prostate cancer described in Greenberg *et al.*, 1995, Proc. Natl. Acad. Sci. USA. 92: 3439-3443, of which amount is herein incorporated by reference).

To conclude "during the course" of a treatment that this treatment is inefficient, the skilled person will of course choose to analyse the amounts of said tumour markers after the treatment has being applied for a time that is sufficiently long for this particular treatment to have a reasonable expectation of showing a significant anti-tumour activity on the particular animal being tentatively treated. Alternatively, or complementarily, the skilled person may choose to repeat this marker amount analysis several times during the course of the treatment, thereby determining the effects of the treatment as a function of time of application, so as to decide whether said treatment is capable of developing beneficial effects in the body of said animal, or whether it is very much unlikely to do so. Determining the minimum duration that is necessary to reach a reliable conclusion with a minimum of deleterious side effects is a matter of experimental or clinical optimisation that belongs to the common general expertise of the physician or clinician.

The invention also provides with shaper methods to determine whether an applied treatment is or will be highly efficient against a tumour which is present but not developing or spreading, or whether it is or will be inefficient against such a tumour. These methods are particularly reliable for adenomas and adenocarcinomas, and more particularly for thyroid adenomas and adenocarcinomas.
One such sharper method for determining whether an applied treatment is or will be highly efficient against a tumour which is present but not developing or spreading, or whether it is or will be inefficient against such a tumour, comprises the following step:
- measuring at least one cellular amount selected from the group consisting of the cellular amount of FADD proteins which is present in cells that are representative of said tumour during the course or at the end of said treatment, and the cellular amount of phosphorylated p38-MAPKs which is present in cells that are representative of said tumour during the course or at the end of said treatment,
wherein said treatment is determined as efficient against said tumour, when said measured cellular amount(s) is(are) significantly superior to the (respective) control cellular amount(s) which is(are) observed in untreated but otherwise comparable control cells,
wherein said treatment is determined as inefficient against said tumour, when said measured cellular amount(s) is(are) not significantly superior to said (respective) control cellular amount(s),
and/or the following step:
- measuring the amount of FADD proteins which is present in the extracellular environment of cells which are representative of said tumour during the course or at the end of said treatment,
wherein said treatment is determined as efficient against said tumour, when said measured extracellular FADD amount is significantly inferior to the control extracellular amount of FADD proteins which is observed in the extracellular environment of untreated but otherwise comparable control cells,
wherein said treatment is determined as inefficient against said tumour, when said measured extracellular FADD amount is not significantly inferior to said control extracellular amount of FADD proteins,
provided that the standard normal cellular amount(s) of FADD proteins and/or of phosphorylated p38-MAPKs, respectively, which is(are) observed in non-tumour but otherwise equivalent cells, is(are) significantly different from zero.

To measure said protein amount(s), there can be provided a biological (cellular or extracellular) sample which originates from the organism wherein said tumour is present, or from a representative biological model thereof (wherein a comparable tumour is present) as representative of said tumour, during the course or at the end of said treatment is provided.

Another such sharper method for determining whether an applied treatment is or will be efficient against a tumour which is in a phase of development or of spreading, or whether it is or will be inefficient against such a tumour, comprises the following step:
- measuring at least one cellular amount selected from the group consisting of the cellular amount of FADD proteins which is present in cells that are representative of said tumour during the course or at the end of said treatment, and the cellular amount of phosphorylated p38-MAPKs which is present in cells that are representative of said tumour during the course or at the end of said treatment,
wherein said treatment is determined as efficient against said tumour, when said measured cellular amount(s) is(are) not significantly inferior to the (respective) control cellular amount(s) which is(are) observed in untreated but otherwise comparable control cells,
wherein said treatment is determined as inefficient against said tumour, when said measured cellular amount(s) is(are) significantly inferior to said (respective) control cellular amount(s),
and/or the following steps:
- measuring the amount of FADD proteins which is present in the extracellular environment of cells that are representative of the cells of said tumour during the course or at the end of said treatment,
wherein said treatment is determined as efficient against said tumour, when said measured extracellular FADD amount is not significantly superior to the control extracellular amount of FADD proteins which is observed in the extracellular environment of untreated but otherwise comparable control cells,
wherein said treatment is determined as inefficient against said tumour, when said measured extracellular FADD amount is significantly superior to said control extracellular amount of FADD proteins,
provided that the standard normal cellular amount(s) of FADD proteins and/or of phosphorylated p38-MAPKs, respectively, which is(are) observed in non-tumour but otherwise equivalent cells, is(are) significantly different from zero.

To measure said protein amount(s), there can be provided a biological (cellular or extracellular) sample which originates from the organism wherein said tumour is present, or from a representative biological model thereof (wherein a comparable tumour is present) as representative of said tumour, during the course or at the end of said treatment is provided.

Given that when an anti-tumour treatment is efficient, the treated organism is said to be susceptible to this treatment, and that when an anti-tumour treatment is or becomes inefficient, the tentatively treated organism is said to be or to become resistant to this treatment, the method of the invention to determine whether a treatment is efficient or not can also be considered as a method to determine whether an animal, wherein a tumour is present, or in development or in spreading, is susceptible to a tentative anti-tumour treatment, or whether this animal is resistant to this tentative treatment.
Quite similarly, given that the degree of resistance, or of susceptibility of the animal which is subjected to anti-tumour treatment is said to reflect the degree of aggressiveness of the tumour disease, the method to determine according to the invention whether a treatment is efficient or not can also be considered as a method to determine whether the tumour development has a high degree of aggressiveness (resistance to applied treatment, inefficient treatment), or whether it has a low degree of aggressiveness (susceptibility to applied treatment, efficient treatment).

These methods of the invention therefore allow to adjust and to optimise the applied treatment as necessary, and most importantly avoid treatments the continuation of which will anyhow not succeed in treating or arresting the tumour, and may even be deleterious to the patient.

The application also relates to a method to determine whether a compound can be useful as an anti-tumour active compound to treat or arrest the growth of a tumour which is present or in development or in spreading, which comprises the following steps:
- contacting said compound with cells representative of said tumour,
- measuring at least one cellular amount selected from the group consisting of the cellular amount of FADD proteins which is present in said contacted cells, and the cellular amount of phosphorylated p38-MAPKs which is present in said contacted cells,
wherein said compound is determined as useful as an anti-tumour active compound to treat or arrest the growth of said tumour, when said measured cellular amount(s) is(are) not significantly inferior to the (respective) standard normal cellular amount(s) which is(are) observed in non-tumour but otherwise equivalent cells,
and/or the following steps:
- contacting said compound with cells representative of said tumour,
- measuring the extracellular amount of FADD proteins which is present in the extracellular environment of said contacted cells,
wherein said compound is determined as useful as an anti-tumour active compound to treat or arrest the growth of said tumour, when said measured extracellular amount is either not significantly different from zero and/or not significantly superior to the standard normal extracellular amount of FADD proteins which is observed in non-tumour but otherwise equivalent cells,
provided that the standard normal cellular amount(s) of FADD proteins and/or of phosphorylated p38-MAPKs, respectively, which is(are) observed in non-tumour but otherwise equivalent cells, is(are) significantly different from zero.

More particularly, the application relates to sharper methods to determine the usefulness of a compound as an anti-tumour active compound, which are particularly reliable for adenomas and adenocarcinomas, and more particularly for thyroid adenomas and adenocarcinomas.

One such method to determine whether a compound can or cannot be useful as an anti-tumour active compound against a tumour which is present but not developing or spreadingcomprises the following steps:
- contacting said compound with cells representative of said tumour,
- measuring at least one cellular amount selected from the group consisting of the cellular amount of FADD proteins which is present in said contacted cells, and the cellular amount of phosphorylated p38-MAPKs which is present in said contacted cells,
wherein said compound is determined as useful as an anti-tumour active compound against said tumour, when said measured cellular amount(s) is(are) significantly superior to the (respective) control cellular amount(s) which is(are) observed in uncontacted but otherwise comparable control cells,
wherein said compound is determined as useless as an anti-tumour active compound against said tumour, when said measured cellular amount(s) is(are) not significantly superior to said (respective) control cellular amount(s),
and/or the following steps:
- contacting said compound with cells representative of said tumour,
- measuring the amount of FADD proteins which is present in the extracellular environment of said contacted cells,
wherein said compound is determined as useful as an anti-tumour active compound against said tumour, when said measured extracellular FADD amount is significantly inferior to the control extracellular amount of FADD proteins which is observed in the extracellular environment of uncontacted but otherwise comparable control cells,
wherein said compound is determined as useless as an anti-tumour active compound against said tumour, when said measured extracellular FADD amount is not significantly inferior to said control extracellular amount of FADD proteins,
provided that the standard normal cellular amount(s) of FADD proteins and/or of phosphorylated p38-MAPKs, respectively, which is(are) observed in non-tumour but otherwise equivalent cells, is(are) significantly different from zero.

Another such method to determine whether a compound can or cannot be useful an anti-tumour active compound against a tumour which is in a phase of development or of spreading, which comprises the following steps:
- contacting said compound with cells representative of said tumour,
- measuring at least one cellular amount selected from the group consisting of the cellular amount of FADD proteins which is present in said contacted cells, and the cellular amount of phosphorylated p38-MAPKs which is present in said contacted cells,
wherein said compound is determined as useful as an anti-tumour active compound against said tumour, when said measured cellular amount(s) is(are) not significantly inferior to the (respective) control cellular amount(s) which is(are) observed in uncontacted but otherwise comparable control cells,
wherein said compound is determined as useless as an anti-tumour active compound against said tumour, when said measured cellular amount(s) is(are) significantly inferior to said (respective) control cellular amount(s),
and/or the following steps:
- contacting said compound with cells representative of said tumour,
- measuring the amount of FADD proteins which is present in the extracellular environment of said contacted cells,
wherein said compound is determined as useful as an anti-tumour active compound against said tumour, when said measured extracellular FADD amount is not significantly superior to the control extracellular amount of FADD proteins which is observed in the extracellular environment of uncontacted but otherwise comparable control cells,
wherein said compound is determined as useless as an anti-tumour active compound against said tumour, when said measured extracellular FADD amount is significantly superior to said control extracellular amount of FADD proteins, provided that the standard normal cellular amount(s) of FADD proteins and/or of phosphorylated p38-MAPKs, respectively, which is(are) observed in non-tumour but otherwise equivalent cells, is(are) significantly different from zero.

The kit for determining absence/presence and/or regression/development of a tumour, as above-defined, also constitute a kit for determining whether a treatment is or will be efficient against a tumour, or whether a compound can or cannot be useful as an anti-tumour active compound against a tumour. Such kits are hence encompassed by the present application.

By "anti-tumour activity" or "pro-tumour activity", it is herein meant an activity that is unfavourable or favourable to the development of a tumour in an organism, respectively, and notably a tumour-inhibiting activity or a tumour-inducing activity in a tumour-free organism, respectively.

As the inventors have demonstrated that several non-tumour primary cell cultures (such as non-tumour thyroid follicular cells (TFC)) or established cell lines loose FADD expression and/or phosphorylation of p38-MAPK under normal standard *in vitro* conditions, without being contacted with a tumour-inducing agent, the application also relates, according to a still further aspect of the invention, to the use of such an *in vitro* culture or established cell line, as an *in vitro* model to determine in the absence of any tumour-inducing agent whether a compound has an anti-tumour activity. Such a use is particularly reliable for adenomas and adenocarcinomas, and more particularly for thyroid adenomas and adenocarcinomas.
The application more particularly relates to a method for determining whether a compound can have an anti-tumour activity in an organism such as an animal, notably a mammal, advantageously a human person, without having to use any tumour-inducing agent, said method being particularly reliable for adenomas and adenocarcinomas, and more particularly for thyroid adenomas and adenocarcinomas.

An example of such a method is characterised in that it comprises the following steps:
- non-tumour thyroid follicular cells (TFC) are placed for *in vitro* growth under conditions of substrate composition, of temperature and of time which enable the adhesion and confluence of said TFC,
- said compound is placed in contact with said culture,
- at least one cellular amount selected from the group consisting of the cellular amount of FADD proteins and the cellular amount of phosphorylated p38-MAPKs is measured in said TFC after said contact with said compound,
wherein said compound is determined as capable of having an anti-tumour activity in said organism, when the measured cellular amount(s) is(are) either significantly superior to the (respective) control cellular amount(s) which is(are) observed in comparable *in* vitro-grown TFC but in the absence of contact with said compound, or not significantly different from the standard (respective) normal cellular amount(s) which is(are) measured in *ex vivo* non-tumour TFC,
and/or the following steps:
- non-tumour thyroid follicular cells (TFC) are placed for *in vitro* growth under conditions of substrate composition, of temperature and of time which enable the adhesion and confluence of said TFC,
- said compound is placed in contact with said culture,
- the extracellular amount of FADD proteins is measured in the extracellular environment of said TFC after said contact with said compound,
wherein said compound is determined as capable of having an anti-tumour activity in said organism, when the measured extracellular FADD amount is either significantly inferior to the control extracellular FADD amount which is observed in the extracellular environment of comparable *in* vitro-grown TFC but in the absence of contact with said compound, or not significantly different from the standard normal extracellular FADD amount which is measured in the extracellular environment of *ex vivo* non-tumour TFC.

Said non-tumour TFC can be collected from any mammal including human persons, and are preferably collected from the same animal variety as the one for which the determination has to be made.
The contact duration which is necessary for said compound to show whether it has or not an effect of the amount of FADD proteins and/or in phosphorylated p38-MAPK of course depends from the particular compound being tested. The minimum duration which is *a priori* necessary for obtaining significant results with a given compound usually belongs to the skilled person's average expertise. Alternatively, or complementary, the skilled person can choose to measure said at least one amount as a function of time until said TFC stop growing.
Said non-tumour TFC intended for said *in vitro* culture may be submitted to a collagenase H treatment before being *in vitro* cultured.
Appropriate conditions for said in vitro growth of TFC notably, and strikingly, include normal standard culture conditions, such as 5-6 days at 37°C on RPMI 1640 medium supplemented with 5% foetal bovine serum.
Examples of candidate compounds notably include modulators of the MAP kinase pathways (thyroid tumours).

The application also relates to a kit to determine in the absence of any tumour-inducing agent whether a compound can have an anti-tumour activity in an organism, characterised in that it comprises:
- non-tumour thyroid follicular cells (TFC), and
- at least one anti-FADD and/or anti-phospho-p38-MAPK compound.
   As non-tumour TFC grown *in vitro* loose FADD expression and p38-MAPK phosphorylation, the method, kit and use of the invention have the advantage of not requiring any tumour-inducing agent for their implementation.

According to the present invention, a method to identify an anti-tumour active compound, among candidate compounds, comprises the selection of said anti-tumour active compound by screening for a compound that prevents FADD proteins from being released from tumoural cells, or from *in vitro* cultured thyroid follicular cells. Examples of candidate compounds notably comprise:
- inhibitors of cellular movements which are associated with actin filaments, such as cytochalasine and latrunculine,
- inhibitors of cellular movements which are associated with the Golgi apparatus, such as brefeldine A,
- inhibitors of vesicle transport which is dependent of ATP, such as N-ethylmaleimide,
- modulators of the MAP kinase pathways.

According to a further aspect of the invention, the application also relates to means for determining the danger rating a compound may have *in vivo* in terms of tumour and notably of cancer. These means are particularly reliable for adenomas and adenocarcinomas, and more particularly for thyroid adenomas and adenocarcinomas.
The application thus relates to a method for assessing whether a compound has a high or a low probability of having a tumour-inducing activity in an organism, such as an animal, and advantageously a mammal, such as a human person. It is characterized in that it comprises the following steps:
- said compound is placed into contact with cells of said organism, or with cells of a representative biological model thereof,
- at least one cellular amount selected from the group consisting of the cellular amount of FADD proteins and the cellular amount of phosphorylated p38-MAPKs is measured in said contacted cells, and
wherein said compound is determined as having a high probability of having a pro-tumour activity in said organism, when said measured cellular amount(s) is(are) significantly inferior to the standard (respective) normal cellular amount(s) which is(are) observed in non-tumour and uncontacted but otherwise equivalent cells,
wherein said compound is determined as having a low probability of having a pro-tumour activity in said organism, when said measured cellular amount(s) is(are) not significantly inferior to said standard (respective) normal cellular amount(s),
and/or the following steps:
- said compound is placed into contact with said cells of said organism, or with cells of a representative biological model thereof,
- the extracellular amount of FADD proteins which is present in the extracellular environment of the cells of said organism or representative biological model thereof is measured in said contacted cells,
wherein said compound is determined as having a high probability of having a pro-tumour activity in said organism, when said measured extracellular amount is significantly superior to the standard normal extracellular amount of FADD proteins which is observed in the extracellular environment of non-tumour and uncontacted but otherwise equivalent cells,
wherein said compound is determined as having a low probability of having a pro-tumour activity in said organism, when said measured extracellular amount is not significantly superior to said standard normal extracellular amount of FADD proteins,
provided that the standard normal cellular amount(s) of FADD proteins and/or of phosphorylated p38-MAPKs, respectively, which is(are) observed in non-tumour but otherwise equivalent cells, is(are) significantly different from zero.

This method is particularly useful for any animal and notably for mammals, and more particularly for human persons. For healthcare and safety purposes, a tumour-free representative biological thereof is preferably used for the implementation of the method. Such a representative biological model thereof can be an *in vivo* model such as *e.g* a mouse, a monkey, a rabbit, a dog, when said animal is a human person, or can be an *in vitro* model, such as a culture obtainable by culture of cells or tissue collected from said animal as representative of the susceptibility of said animal to develop a tumour, provided that the amount of FADD proteins and/or in phosphorylated p38-MAPKs does not decrease with time in this culture (which will be notably the case for *in vitro* standard culture of non-tumour thyrocytes).
Cultures should be placed and kept under conditions of temperature, medium composition, atmosphere (CO₂ amount, O₂ amount), humidity, light, which are appropriate for them to keep alive or to grow when they are not placed in contact with any candidate compound (control culture). Such cultures should not be thyrocyte cultures, as the inventors have observed that cultures of non-tumour thyrocytes under standard normal conditions induce a decrease in the amount of FADD proteins and phosphorylated p38-MAPKs ; it implies that the standard (respective) normal amount(s) of markers of the invention gradually tends(tend), for such thyrocyte culture, toward a value which is(are) not significantly different from zero. Such cultures may however be, for example, hepatocyte cultures. The best way to proceed according to the invention is to I check that the amount of markers of the invention does not decrease with time for the particular culture that is chosen by the skilled person. Examples of candidate compounds to be tested notably include inhibitors of kinase functions.

The application also relates to a kit to assess whether a compound has a high or a low probability of having a pro-tumour activity in an organism, and a pro-adenoma or pro-adenocarcinoma activity, and more particularly a pro-thyroid adenoma or a pro-thyroid adenocarcinoma activity. Such a kit comprises:
- at least one anti-FADD and/or anti-phospho-p38-MAPK specific compound, and optionally a tumour-free biological model, such as a cell culture, and
- appropriate instructions for using a FADD protein and/or a phosphorylated p38-MAPK as pro-tumour indicator(s).

The application more generally relates to the use of a compound which is capable of specifically binding to FADD proteins and/or to phosphorylated p38-MAPKs, or of a biological unit which is capable of producing such a compound, for determining on a representative biological sample, whether there is or not a tumour in an organism, and/or for the prognosis of the resistance of a tumour to an anti-tumour chemotherapy, and/or for determining whether an applied anti-tumour treatment is or will be efficient or not in an organism, and/or for determining whether a compound can have an anti-tumour activity in an organism, and/or for assessing whether a compound has a high or a low probability of having a pro-tumour activity in an organism.

According to the present application, there is therefore provided a method to produce a product useful for determining whether there is or not a tumour in an organism, and/or for the prognosis of resistance of a tumour to an anti-tumour chemotherapy, and/or for determining whether an applied anti-tumour treatment is or will be efficient or not in an organism, and/or for determining whether a compound can have an anti-tumour activity in an organism, and/or for assessing whether a compound has a high or a low probability of having a pro-tumour activity in an organism, characterised in that it comprises the step of producing said product by production of a compound which specifically binds to FADD proteins and/or to phosphorylated p38-MAPKs, optionally followed by binding a detection label to this anti-FADD and/or anti-phosphorylated p38-MAPK specific compound.

To detect whether FADD proteins and/or phosphorylated p38-MAPK proteins are present or absent in a sample, as well as to possibly detect at which amount they are respectively present, the skilled person can use any standard procedure for protein detection and quantification (see e.g. Current Protocols in Protein Science Edited by Coligan John E., & *al.* 2001. John Wiley & sons Inc.; and Current Protocols in Immunology. Edited by Coligan John E., & *al.* 2001. John Wiley & Sons Inc., of which amounts are herein incorporated by reference). Such detection is usually made by using a compound that is capable of specifically binding to FADD proteins and/or to phosphorylated p38-MAPK.
The invention therefore relates to the use of a compound that is capable of specifically binding to FADD proteins and/or to phosphorylated p38-MAPKs, or of a biological unit which is capable of producing such a compound, for determining whether there is or not a tumour in an organism, and/or whether an anti-tumour treatment is, or will be, efficient or not in an organism. It more particularly encompasses a method and a kit for determining whether there is or not a tumour in an organism, and/or whether an anti-tumour treatment is or will be efficient or not in an organism.
The method to produce according to the invention a product useful for determining whether there is or not a tumour in an organism, and/or whether an anti-tumour treatment is, or will be, efficient or not in an organism is characterised in that it comprises the step of obtaining said product by production of a compound which specifically binds to FADD and/or to phosphorylated p38-MAPK, possibly followed by binding a detection label to this anti-FADD and/or anti-phosphorylated p38-MAPK specific compound.
The kit of the invention for determining whether there is or not a tumour in an organism, and/or whether an anti-tumour treatment is, or will be, efficient or not in an organism is characterised in that it comprises:
- at least one compound which is capable of specifically binding to FADD proteins and/or to phosphorylated p38-MAPK, or at least one biological unit which is capable of producing such a compound, this at least one compound or biological unit being possibly contained in a physiological buffer, and/or possibly bound to a solid support, and
- appropriate instructions for using FADD proteins and/or phosphorylated p38-MAPK as marker of the absence of an *in vivo* tumour.

By a compound which is capable of specifically binding to FADD and/or phosphorylated MAPK, it is herein meant that this compound can show with its target(s) a binding reaction which is of the antibody-antigen type, when this compound is placed under conditions appropriate for antibody-antigen type reactions, whereas when placed under the same conditions, this compound does not show a significant reaction of the antibody-antigen type with those other non-target molecular compounds of an animal cell, preferably of a mammal cell, most preferably of a human cell. Such a specific compound does advantageously not cross-react with other proteins of the MAPKinase family and with proteins having a death domain which is homologue (homology of 80% or greater, preferably of 70% or greater, most preferably of 60% or greater) to the death domain of the FADD protein.

Examples of conditions appropriate for antibody-antigen type reactions include standard assay conditions for protein detection and/or quantification, *e.g.* standard ELISA conditions, immuno(histo)chemistry conditions, standard Western/Dot/Slot blot conditions (see the below examples for illustrative immunochemistry and Western blot standard procedures, see also Harlow *et al.* ("*Antibodies - A Laboratory Manual*", Cold Spring Harbor Laboratory, 1988, edited by Edward Harlow and David Lane of which entire amount is herein incorporated by reference ; Les Editions Inserm. 1987. Techniques en Immunologie. Techniques Immuno-enzymatiques. Th., Terninck & S., Avrameas. ; Current Protocols in Protein Science Edited by Coligan John E., & al. 2001. John Wiley & Sons Inc.; and Current Protocols in Immunology. Edited by Coligan John E., & al. 2001. John Wiley & Sons Inc. of which entire amounts are herein incorporated by reference).

For said detection and/or quantification, said sample may be immobilized onto a solid support such as a membrane or a chip, or may be frozen and cut into sections.

Alternatively, the skilled person in the art may use FACS analysis to detect whether said binding compound finds a target in the analysed sample (see e.g. Current Protocols in Cytometry. Edited by J. Paul, Robinson. 2001. John Wiley & Sons Inc. of which entire amount is herein incorporated by reference). For instance, permeabilization of the cells to be analysed could be performed and used for intracellular detection of target antigen.

Such specific compounds notably comprise antibodies specific for FADD proteins and/or against phosphorylated p38-MAPK. Said biotechnological unit can then be an hybridoma which is capable of producing such an antibody. This antibody may be monovalent or divalent, polyclonal or monoclonal. Examples of such antibodies include the polyclonal goat anti-FADD IgG commercialised by Santa Cruz Biotechnology with the product reference MC19, and the anti-phospho-p38-MAPK(thr180/tyrl82) antibody commercialised by Cell Signalling Technology with the polyclonal goat anti-FADD IgG (M-19 - previously referenced under ref. MC-19 -) Santa Cruz Biotechnology ref. sc-6036; polyclonal goat anti-FADD IgG (N-18) Santa Cruz Biotechnology ref. sc-1172; polyclonal rabbit anti-FADD IgG (125-140) Calbiochem ref. 341282; polyclonal rabbit anti-phospho-p38-MAPK(thr180/tyrl82) Cell Signalling Technology ref. 9211.

Other such antibodies can be produced by the person skilled person in the art according to standard methods in the field, described, for example, by Harlow *et al.* ("*Antibodies - A Laboratory Manual*", Cold Spring Harbor Laboratory, 1988, edited by Edward Harlow and David Lane), of which entire amount is herein incorporated by reference.

Such an anti-FADD and/or anti-phospho-p38-MAPK specific antibody may also be a monoclonal antibody. For diagnosis applications, monoclonal antibodies are preferred. Appropriate monoclonal antibodies can be produced by conventional techniques, such as the one described by Köhler and Milstein, 1975 (Nature 256:495-497 *"Continuous cultures of fused cells secreting antibodies of predefined specificity*"), of which entire amount is herein incorporated by reference. Examples of appropriate monoclonal antibodies include for example the anti-FADD mAbs commercialised by MBL International Corporation (200 Dexter Ave. Suite D Watertown, MA 02472, USA) [product references M033-3 (1F7, mouse IgG1) and MO35-3 (4G3, mouse IgG1)].

Such a compound may also be a fragment or a derivative of such an polyclonal or monoclonal antibody or diabody, provided that this fragment or derivative has retained an anti-FADD and/or auto-phospho-p38-MAPK specificity, as above defined. Examples of such fragments notably include F(ab')₂, Fab, Fv fragments. Examples of such derivatives notably include monovalent and divalent single chain antibodies (scFv), disulfide bond-stabilised Fv antibody fragments (dsFvs), bispecific dsFv-dsFv' constructs.

Such an anti-FADD and/or anti-phospho-p38-MAPK specific compound is thus obtainable, for example, by:
i.
   - immunization of an animal against a FADD protein and/or a phosphorylated p38-MAPK,
   - collection of the antibodies thus produced, and by
   - isolation of those antibodies which compete with anti-FADD and/or anti-phospho-p38-MAPK antibodies, such as anti-mouse FADD (M-19) Santa Cruz Biotechnology ref. sc-6036 and/or anti-human FADD (125-140) Calbiochem ref. 341282 and/or anti-mouse and human phospho-p38-MAPK Cell Signalling Technology ref. 9211, for binding to FADD and/or phosphorylated p38-MAPK, and do not cross-react with other endogenous proteins, as above described. or by:
ii.
   - isolation of the Fv, Fab or F(ab')₂ fragments of such an anti-FADD and/or anti-phospho-p38-MAPK specific antibody(ies),
      or by:
iii.
   - grafting the CDRs fragments of such an anti-FADD and/or anti-phospho-p38-MAPK specific antibody(ies) onto a molecular structure which has the structure of an antibody backbone,
      or by:
iv.
   - operably cloning in a host cell, such as *E. coli,* the polynucleotide sequence of such an anti-FADD and/or anti-phospho-p38-MAPK specific antibody(ies), or of such a Fv, Fab or F(ab')₂ fragment, or of such a grafted molecular structure, and recovering the product produced by this host cell *via* the transcription and translation of said polynucleotide sequence.

Said kit of the invention for determining whether there is or not a tumour in an animal, and/or whether a tentative anti-tumour treatment is efficient or not may also comprise detection labels appropriate for detection of possible binding of said at least one binding compound to its target.
Examples of such detection labels include for example fluorescent labels or radioactive labels which may be coupled to the binding compound.
Other examples include secondary antibodies which can bind to said binding compound, such as horseradish peroxydase conjugated antibodies which enable to visualize such a binding after incubation on an ECL substrate (Amersham Pharmacia Biotech), or biotinylated conjugated secondary antibodies (Vector) which enable to visualize such a binding after incubation with phosphatase alcaline conjugated streptavidin (Amersham Life Science) on Fast-red substrate (Acros organics).

In all the methods, kits and use of the invention, the cellular amount of FasL proteins (of which expression is gained with the development of a tumour) is advantageously measured in addition to detection of the cellular amount of FADD proteins and/or phosphorylated p38-MAPK, so as to determine whether this amount increases compared to the standard normal FasL cellular amount that is observed in non-tumour but otherwise equivalent conditions, thereby confirming that a tumour is present or in development in the organism, and/or that the tentative anti-tumour treatment is inefficient, and/or that the tested compound has a high probability of having *in vivo* tumour-inducing activities, and/or that the compound has no anti-tumour or anti-mitotic *in vivo* activity.

The following examples are provided for the purposes of illustration, and are not intended to limit the scope of the present invention. They make various embodiments and advantages of the invention apparent to the skilled person. In these examples, reference is made to the following figures:
- Figures 1A and 1B illustrate that Fas ligand expression is acquired during tumour development. Figure 1A shows representative results of semi-quantitative RT-PCR of FasL mRNA from thyroid glands of gsp transgenic mice at various stages of tumour development, while Figure 1B shows the corresponding analysis for FasL proteins on Western blot. Amplified fragment and protein sizes are indicated. Differential FasL expression by the two lobes of the same adenoma is shown in lanes 7 and 8,

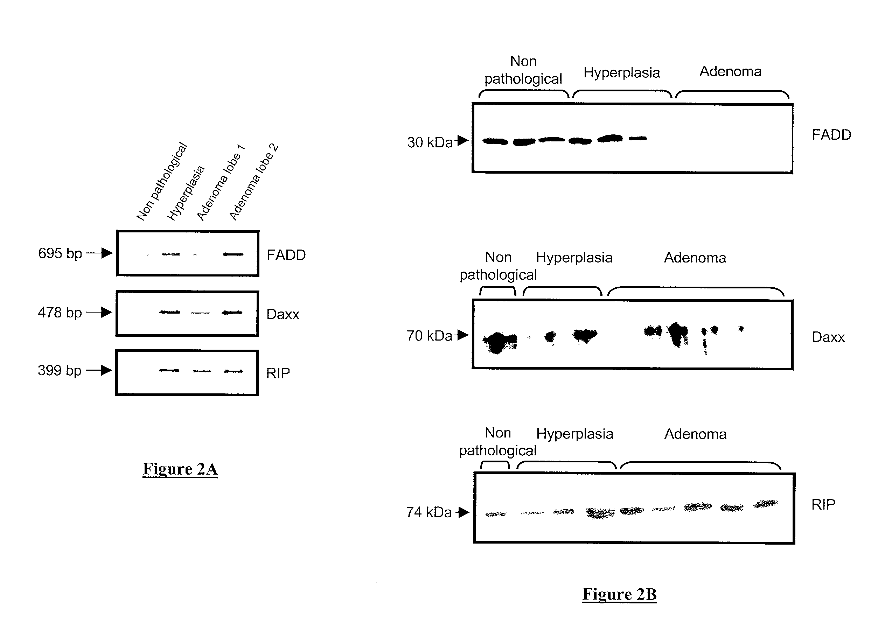

- Figures 2A and 2B illustrate that the adapter protein FADD is lost during tumour development. Figure 2A shows semi-quantitative RT-PCR analysis of FADD, Daxx and RIP mRNAs expression by thyroid glands from gsp transgenic mice at various stages of tumour development. The expression of the adapters mRNAs in the two lobes of the same adenoma is shown in lanes 3 and 4. Amplified fragments size is indicated. Figure 2B shows Western blot analysis of the three adapter molecules by thyroid glands from gsp transgenic mice at various stages of tumour development (FADD = panel 1 ; Daxx = panel 2 ; RIP = panel 3). Proteins size is indicated,
- Figure 3 illustrates that the p38-MAPK pathway is inhibited during tumour development, by showing Western blot analysis of total (upper panel) and active phosphorylated (lower panel) forms of p38-MAPK expression by thyroids glands from gsp transgenic mice at various stages of tumour development,

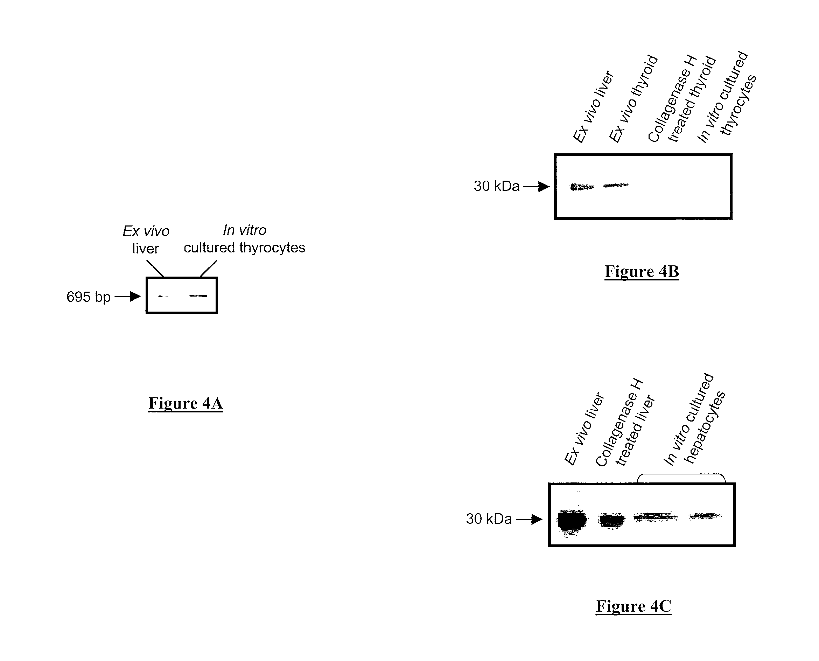

- Figures 4A, 4B and 4C illustrate that *in vitro* cultured thyrocytes do not express FADD protein. Figure 4A shows semi-quantitative RT-PCR analysis of FADD mRNA expression by *ex vivo* liver and *in vitro* culture of thyroid cells from normal non transgenic mice. Figure 4B and 4C show Western blot analysis of FADD protein expression by *ex vivo,* collagenase H treated and *in vitro* cultured thyrocytes (Figure 4B) or hepatocytes (Figure 4C) from normal non transgenic mice. Results of two independent *in vitro* cultures of hepatocytes are shown in lanes 3 and 4. Amplified fragment and protein sizes are indicated.
- Figure 5 illustrates that Fas signalling in absence of FADD increases TFC growth. Normal thyrocytes were cultured with or without clone Jo2 anti-Fas antibody for 24h to 120h exposure. Proliferation of thyrocytes was determined every day until confluence by adding [³H] thymidine for the last 12h of culture. Data represent percentage of proliferation increase of anti-Fas versus control cultures. Each point is mean cpm of six wells cultures. Value for proliferation of control TFC varied from 343 cpm to 1382 cpm. Value for proliferation of anti-Fas treated TFC varied from 392 cpm to 1507 cpm.
- Figure 6 is a schematic representation of the mechanisms by which the Fas pathway is regulated,
- Figure 7 shows human and *Mus musculus* FADD and p38-MAPK aminoacid sequences,
- Figure 8 shows a schematic definition of FADD labelling detected and observed by immunohistochemistry,
- Figure 9 shows a Western blot analysis of the presence or absence of FADD proteins in tumoral cells from leukemic patients,
- Figure 10 shows results of ELISA analysis for FADD protein in serum samples collected from healthy human beings (control),
- Figures 11A and 11B show results of ELISA analysis for FADD protein in serum samples collected from CML patients,
- Figures 12A, 12B and 12C show results of ELISA analysis for FADD protein in serum samples collected from AML patients,
- Figure 13 shows results of ELISA analysis for FADD protein in serum samples collected from ALL patients,
- Figure 14 shows a comparative representation of the results of ELISA analysis for FADD protein in serum sample of healthy/CML/AML/ALL patients,
- Figures 15A and 15B show a Western blot analysis of the kinetic of FADD release from cells of in vitro cultured normal thyroid lobes,
- Figures 16A and 16B show Western blot analysis of tentative treatment to arrest FADD release from the cells of in vitro cultured normal thyroid lobes,
- Figures 17A and 17B show mass spectrometry analysis of supernatants of lh-old culture of normal thyroid lobes (SN+: in the presence of 2-mercaptoethanol ; SN-: in the absence of 2-mercaptoethanol): Figure 17A shows direct analysis of a 1h culture supernatant (% of peak intensity as a function of MW in Da), Figure 17B shows a similar analysis performed on a supernatant that has been treated on a C4 column to concentrate and eliminate the salts thereof.

### EXAMPLE 1: when a tumour develops in a thyroid adenoma/adenocarcinoma non-human mammal model, FADD expression is lost (but not FADD mRNA), and there is a correlated decrease in phosphorylated p38-MAPK.

Due to the multiple possible artefacts and to the controversy existing on the detection of some of the molecules of the Fas cascade, we made the choice of investigating the presence of a large array of the signalling molecules of the Fas cascade in an *in vivo* tumour model, and to combine three different techniques (semi-quantitative RT-PCR, western blot and immunohistochemistry) to detect and quantify molecules both at the mRNA and protein levels and to localize protein expressing cells.
The *in vivo* tumour model we selected is a gsp transgenic mouse. In thyroid gland, guanine nucleotide stimulatory factor (Gs) activates adenylate cyclase in response to thyrotropin stimulation, leading to cyclic AMP level elevation and subsequent thyroid cells proliferation. Mutations of the α subunit of the Gs factor (gsp mutations), resulting in constitutive activation of adenylate cyclase, are found in approximately 30% of human thyroid toxic adenomas and 10% of human thyroid carcinomas. Transgenic mice expressing a mutant form of Gsα subunit directed to murine thyroid epithelial cells (gsp transgenic mice) are a powerful model for studying human thyroid tumours. These mice have no abnormality up to 8 months of age, and thereafter develop hyperplasia followed by hyperfunctioning thyroid adenomas (Michiels, 1994, PNAS Vol. 91 pp. 10488-10492), or adenocarcinomas depending from the genetic background of the mice.

Our results show that normal TFC of non transgenic (NTG) mice, like non pathological gland of transgenic mice, did not express FasL molecule. By contrast, FasL expression was acquired during tumour development with a higher expression in adenoma/adenocarcinoma than in hyperplastic thyroids. This up-regulation of FasL on tumour cells was correlated with a down-regulation of molecules of the Fas signalling pathway. Among the three adapters for Fas receptor, only FADD was highly regulated. Particularly, we found that although FADD mRNA was present, FADD protein expression was very weak or completely lost in adenoma/adenocarcinoma. Furthermore, by using an *in vitro* model of thyrocyte culture we found that, in the absence of FADD, Fas-signalling resulted in faster growth of thyrocytes apparently through a particular Daxx signalling.

### Materials and Methods

*Mice.* The gsp transgenic mice were described elsewhere (Michiels, 1994, reference cited supra). CBA/J female mice were purchased from Iffa Credo (L'Arbresle, France) and were used at 7-10 weeks of age in all experiments. All mice were maintained in standard environmental conditions with free access to food and water, and were allowed to adapt to their environment for one week before the experiments. Adenoma/adenocarcinoma development was monitored by measurement of T₄ and T₃ serum levels.
*Culture of primary thyrocytes.* Thyroid tissues were obtained from control CBA/J female mice. Animals were sacrificed and the two thyroid lobes cut off after intra-cardiac puncture to eliminate contaminating leukocytes. Collected lobes were digested in a 1.5mg/ml collagenase H (Boehringer Mannheim) solution for 30 minutes at 37°C. Thyroid cells were then cultured at 37°C in RPMI 1640 medium (Life Technologies) supplemented with 5% fetal bovine serum, 100U/ml penicillin (Life Technologies), 100µg/ml streptomycin (Life Technologies) and 5.10⁻⁵ 2-mercaptoethanol (complete culture medium). Non adherent cells were removed by washing to obtain TFC pure cultures. Cells reached sub-confluence after 5-6 days of culture.
*Culture of primary hepatocytes.* BALB/c hepatocytes were prepared according to Mazier *et al.* 1985 (Science Vol. 227 pp. 440-442) with minor modifications. Briefly, cells were isolated by perfusion of the liver fragments with collagenase (Boehringer Mannheim, Germany) following purification in a 60% Percoll gradient (Pharmacia Biotech, Uppsala, Sweden). Purified hepatocytes showed at least 95% of viability and purity assessed by Trypan blue dye exclusion. They were cultured in William's E medium (Gibco, Irvine, UK) supplemented with 5% of FCS (Gibco) and 1% of penicillin-streptomycin solution (100 X stock solution, Gibco) following incubation at 37°C for 24 h under 3.5% CO2 atmosphere.
*Semi-quantitative RT-PCR.* Thyroids of gsp transgenic mice were collected at various stages of tumour development and RNA was isolated from cells using 500µl to lml Tri-Reagent per thyroid (Molecular Research Center). For the *in vitro* culture model, TFC were cultured in 24-well flat bottomed plates (Costar) at approximately 10⁵ cells/well in a final volume of lml. At sub-confluence, thyroid cell cultures were washed with PBS and RNA was isolated from cells using 250µl/well Tri-Reagent. Contaminating DNA was eliminated by DNAse (Ozyme) treatment. Reverse transcription was performed on 2µg total RNA using Superscript II reverse transcriptase (Life Technologies). ADNc (50ng) was then amplified by polymerase chain reaction (PCR) using 0.5U Taq polymerase (Life Technologies). Specific primers used for PCR were: 
FasL (425-bp)
sense 5'-CAGCAGTGCCACTTCATCTTGG-3' (SEQ ID NO:1),
antisense 5'-TTCACTCCAGAGATCAGAGCGG-3' (SEQ ID NO:2),
 
Fas (366-bp)
sense 5'-CTTCTACTGCGATTCTCCTG-3' (SEQ ID NO:3),
antisense 5'-CTTGCCCTCCTTGATGTTAT-3' (SEQ ID NO:4),
 
FADD (695-bp)
sense 5'-TGGATGGGATTGAGGAGAAG-3' (SEQ ID NO:5),
 antisense 5'-AAACCACAGTCCTCACAGGG-3' (SEQ ID NO:6), 
Daxx (478-bp)
sense 5'-CCAACCTACCCCCCAATC-3' (SEQ ID NO:7),
antisense 5'-GCCTCAGAGGCAGTGTTTTC-3' (SEQ ID NO:8),
 
RIP (399-bp)
sense 5'-CAGGAGCAAGAGGTCATTCTG-3' (SEQ ID NO:9),
antisense 5'-TGTTATCAAAGATGGCTTGGTG-3' (SEQ ID NO:10),
 
FLICE (488-bp)
sense 5'-GCTGACCTCAGATGTGAGAGG-3' (SEQ ID NO:11),
antisense 5'-TCCCATTGAAGATGTACACAGC-3' (SEQ ID NO:12),
 
FLIPL (389-bp)
sense 5'-TACAGAGTGAGGCGGTTTGA-3' (SEQ ID NO:13),
antisense 5'-AGCGAAGCCTGGAGAGTATT-3' (SEQ ID NO:14),
 
Bcl2 (290-bp)
sense 5'-CGAGAAGAAGGGAGAATCAC-3' (SEQ ID NO:15),
antisense 5'-CATAAGGCAACCACACCATC-3' (SEQ ID NO:16),
 
Bcl-xl, Bad (361-bp)
sense 5'-AGCCCAGAGTATGTTCCAGA-3' (SEQ ID NO:17),
antisense 5'-CTCAAACTCATCGCTCATCC-3' (SEQ ID NO:18),
 
Bax (273-bp)
sense 5'-TGCTACAGGGTTTCATCCAG-3' (SEQ ID NO:19),
antisense 5'-CAAAGTAGAAGAGGGCAACC-3' (SEQ ID NO:20).
 
βactin [sense 5'-TGGAATCCTGTGGCATCCATGAAAC-3' (SEQ ID NO:21),
antisense 5'-TAAAACGCAGCTCAGTAACAGTCCG-3' (SEQ ID NO:22)] was
used as control.
 was used as control
PCR products were loaded onto a 2% agarose gel and visualized by staining with ethidium bromide. For quantification serial two fold dilution of cDNA were amplified for 25 cycles using ßactin primers. cDNA giving equivalent amount of ßactin were then amplified for 24 to 30 PCR cycles to ascertain that equal amount of cDNA were used.
*Western Blot.* Thyroids of gsp transgenic mice were collected at various stages of tumour development and total proteins were extracted with 200µl to 1ml lysis buffer (10mM Tris-HCl, 150mM NaCl pH7,8, 1%NP40, 1mM PMSF, 1µg/ml aprotinin and 1µg/ml leupeptin) containing protein phosphatase inhibitors (1nM cypermethrin, 30µM dephostatin, 50nM okadaic acid, 20nM tautomycin and 1mM orthovanadate) (Calbiochem). For the *in vitro* culture model, TFC were cultured in 6-well flat bottomed plates (Costar) at approximately 250,000 cells/well in a final volume of 2ml. At sub-confluence, thyroid cell cultures were washed with PBS and total proteins were extracted with 500µl/well of lysis buffer. Samples concentrations were determined using micro BCA protein assay reagent kit (Pierce). Electrophoresis through SDS-polyacrylamide gel was performed using 25µg of total proteins, and then transferred to nitrocellulose (NEN). The membranes were blocked with 5% milk in TBS 0.1% Tween 20 for one hour at room temperature (RT). Specific proteins were detected using specific primary antibodies at appropriate concentration: anti-mouse FasL (rat IgG2a; H11) was manufactured by Alexis Biochemicals; anti-mouse Fas (rabbit IgG; FL-335); FADD (goat IgG; MC19); Daxx (rabbit IgG; M112); RIP (rabbit IgG; H-207); caspase 10 (rabbit IgG; H-131); ASK1 (goat IgG; N-19); SEK1/MKK4 (rabbit IgG; C-20); PTEN (goat IgG; N-19); HSP27 (goat IgG; M-20); Apafl (goat IgG; N-19); and Bc12 (rabbit IgG; Δc21) were all obtained from Santa Cruz Biotechnology; anti-mouse caspase 8 and Bax (both rabbit IgG) were from Chemicon; anti-mouse FLIP (rabbit IgG) was from Euromedex and anti-mouse caspase 3 proform (goat IgG; 46) was from Transduction Laboratories; anti-mouse caspase 2 long and short (mouse IgG1; G310-1248) and Bcl-xl (rabbit IgG) were both from Pharmingen; anti-mouse phospho-SEK1/MKK4 (thr261); SAPK/JNK; phospho-SAPK/JNK (thr183/tyr185); MKK3; phospho-MKK3/MKK6 (ser189/207); p38-MAPK; phospho-p38-MAPK (thr180/tyr182); Akt; phospho-Akt (ser473); and Bad were all rabbit IgG obtained from Cell signalling Technology. After washes with TBS 0.1% Tween 20, membranes were incubated with horseradish peroxydase conjugated secondary antibodies for lh at RT. After washing, the blots were developed using an ECL substrate (Amersham Pharmacia Biotech).
*Immunohistochemistry.* Immunohistochemical staining of 5µm frozen fixed tissue sections was performed using primary antibodies at 10µg/ml for 1h at RT. Biotinylated conjugated secondary antibody (Vector) was followed by phosphatase alcaline conjugated streptavidin (Amersham Life Science). Positive reactivity was revealed by adding Fast-red substrate (Acros organics). Staining positivity and intensity was assessed by two persons by blind evaluation of thyroid specimens.
*Proliferation assay.* TFC were cultured in 96-well flat bottomed plates (Costar) at approximately 125,000 cells/ml in a final volume of 150µl in absence or presence of clone Jo2 anti-Fas antibody (0.5µg/ml, Pharmingen). Proliferation of thyrocytes was determined from 24h to 120h. Thyrocytes were pulsed with 0.5µCi of [³H] thymidine during the last 12h of culture. Results are expressed as mean cpm of six wells cultures.

### Results

The results obtained are summarized in the below table 1:

**Table 1:**

| **mRNA and protein levels of the Fas cascade molecules from normal to pathological conditions** | | | | | | |
|---|---|---|---|---|---|---|
| | Non pathological thyroid | | Hyperplastic thyroid | | Adenoma Adenocarcinoma | |
| | mRNA | Protein | mRNA | Protein | mRNA | Protein |
| Fas Ligand | - | - | + | + | ++ or | ++ or +++ |
| Fas | + | + | + | + | + | + |
| FADD | + | +++ | + | +++ or ++ | + | +/- or - |
| DAXX | + | + | + | + | + | + |
| RIP | + | + | + | + | + | + |
| Caspase 8 | +/- | +/- | +/- | +/- | +/- | +/- |
| FLIP | + | ND | + | ND | + | ND |
| Caspase 10 | ND | + | ND | + | ND | + |
| Pro-Caspase 3 | ND | + | ND | + | ND | + |
| Pro-Caspase 2 Long | ND | ++ | ND | ++ or +++ | ND | ++ or +++ |
| Pro-Caspase 2 Short | ND | - | ND | - | ND | - or +/- |
| Caspase 2 Long Pro | ND | - | ND | - or + | ND - | or + or ++ |
| ASK1 | ND | + | ND | + or ++ | ND | + or ++ |
| SEK1/MKK4 | ND | + | ND | + | ND | + or ++ |
| Phospho-SEK1 | ND | + | ND | +/- | ND | +/- |
| SAPK/JNK | ND | + | ND | + | ND | + |
| Phospho-SAPK/JNK | ND | + (2P) | ND | + (1P) | ND | + (1P) |
| MKK3 | ND | +++ | ND | + | ND | + or +/- |
| Phospho-MKK3/6 | ND | +++ | ND | +++ | ND | +++ |
| P38-MAPK | ND | ++ | ND | +++ | ND | + or +++ |
| Phospho-P38-MAPK | ND | +++ | ND | + or +/- | ND | +/- or - |
| PTEN | ND | + | ND | + | ND | + |
| Akt | ND | + | ND | + | ND | + |
| Phospho-Akt | ND | + | ND | + | ND | + |
| HSP 27 | ND | + | ND | + | ND | + |
| Apaf 1 | ND | + | ND | + | ND | + |
| Bcl2 | + | + | + | + | + | + |
| Bcl-xl | + | + | + | + | + | ++ |
| Bad | + | + | + | + | + | + |
| Bax | + | + | + | + | + | ++ |
| - or +/-: null or very low band intensity, | | | | | | |
| +: low band intensity, | | | | | | |
| ++: medium band intensity, | | | | | | |
| +++: high band intensity. | | | | | | |

### Fas ligand expression is acquired during tumour development.

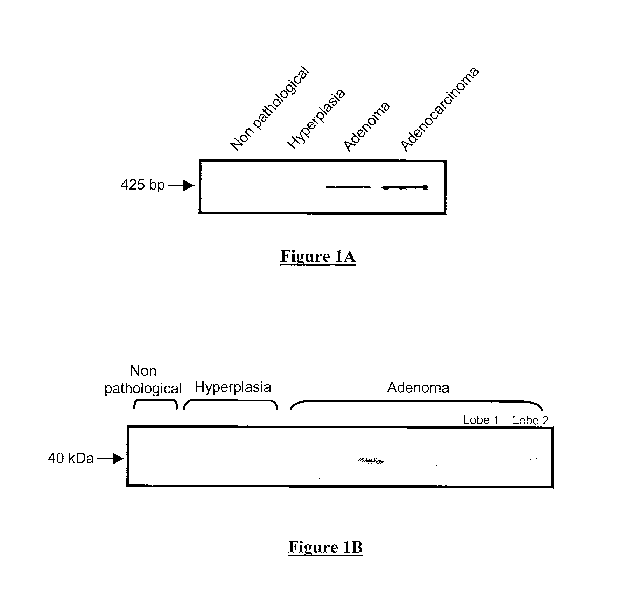

As FasL expression was reported on various cancer cells including human thyroid carcinoma, the presence of this molecule was investigated in mouse thyroid adenomas and adenocarcinomas. Thyroids at various stages of tumour development were analyzed by semi-quantitative RT-PCR and western blot. As previously described, neither FasL mRNA, nor FasL protein could be detected in normal thyroid tissue from NTG mice. In non pathological thyroid glands from gsp transgenic mice, FasL mRNA and protein were absent in all mice tested (see the above Table 1, and see Fig. 1A and 1B respectively) indicating that transgene expression by itself did not induce FasL molecule. By contrast, FasL mRNA and protein could be detected in hyperplastic thyroids and at higher level in thyroid adenomas and adenocarcinomas (Table 1 and Fig. 1A and 1B). The results showed that only diseased lobes were positive for FasL. Moreover, the soluble form of FasL was always detected in adenomas and was expressed stronger than the membrane-bound form of FasL. Those results indicate that thyrocytes acquire FasL expression during tumourigenesis. By contrast, RT-PCR and western blot analysis showed that Fas mRNA and protein were indifferently expressed both in non pathological and pathological thyroids (Table 1).

### Daxx and RIP are continuously expressed during tumour development whereas the adapter protein FADD is lost.

The modulation of the three adapter molecules for the Fas receptor was first investigated by semi-quantitative RT-PCR (Table 1 and Fig. 2A). FADD, Daxx and RIP mRNAs were detectable in thyroids from NTG and gsp transgenic mice, and mRNAs level of expression was not significantly modified during the course of tumour growth. Western blot analysis showed that high level of FADD protein was detected in all non pathological (3/3) and hyperplastic (4/4) glands of gsp mice. Strikingly, FADD protein expression was very weak (2/5) or completely lost (3/5) in adenomas and adenocarcinomas (Table 1 and Fig. 2B panel 1). Immunohistochemistry confirmed those results and showed that FADD protein expression was limited to restricted areas or completely lost in adenomas and adenocarcinomas. By contrast, western blot (Table 1 and Fig. 2B) and immunohistochemistry analysis confirmed that RIP and an approximately 70 kDa form of Daxx proteins were both expressed at all stages of tumour development in gsp transgenic mice. Daxx protein expression was unmodified or moderately decreased in adenomas/adenocarcinomas (Fig. 2B panel 2). Whatever the tumour status of the gland, the 120 kDa form of Daxx could not be detected even in the nucleus . Compared to non pathological thyroid gland, RIP protein expression was slightly increased in adenomas (Fig. 2B panel 3).

### Daxx mediated signal is a main Fas signalling pathway in absence of FADD.

The above results showed that both Daxx and RIP, but not FADD protein, were expressed in thyroid adenoma/adenocarcinoma. The next step was to study the downstream effector proteins of the Daxx and RIP signalling pathways. Western blot analysis of ASK 1 protein, the intermediate between the adapter Daxx and the kinases pathway, showed that this molecule was expressed in thyroids of gsp transgenic mice at all stages of tumour development, and that ASK1 expression strongly increased during tumour growth (Table 1). On the other hand, RIP adapter can induce apoptosis due to caspase 2 activation. Western blot analysis showed that Pro-caspase 2 long (48 kDa) was expressed at high level in all tested thyroids, independently of the tumour status of the gland. By contrast, inhibitory forms of caspase 2 (Pro-caspase 2 short (35 kDa) and/or caspase 2 long-Prodomain (15 kDa) were only expressed in tumour specimen. Although Pro-caspase 2 short (35 kDa) was barely expressed in adenomas samples, high level of caspase 2 long-Pro (15 kDa) was detected (Table 1). The inhibitory 15 kDa form of caspase 2 was detected in 1 out of 2 hyperplastic gland. Those results suggested that although RIP-caspase 2 pathway could be induced during tumour development of gsp transgenic mice, it was not the main Fas-signalling pathway in this situation. Indeed, all adenomas expressed the 48 kDa apoptotic form of caspase 2, but expressed an inhibitory (35 kDa and/or 15 kDa) form of caspase 2 too. Thus, in absence of FADD, Fas signalling seems to act mainly through a Daxx-ASK1 pathway.

### Inhibition of the kinases pathway during tumour development.

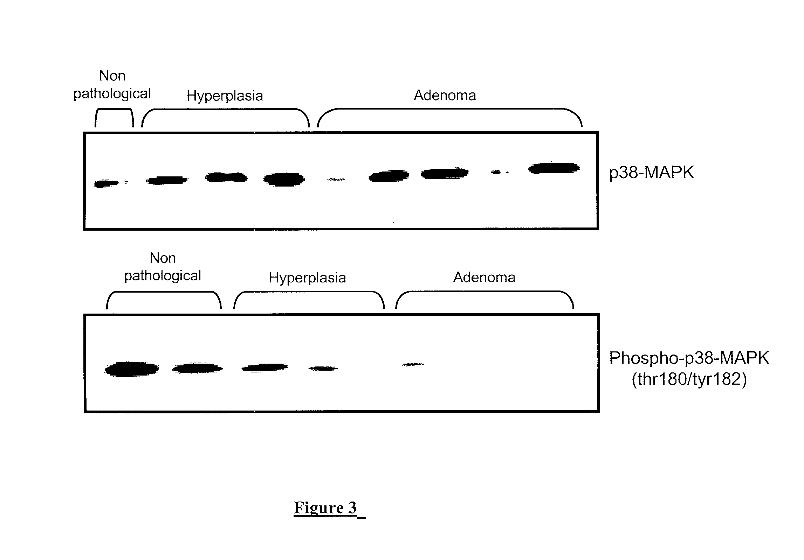

We further investigated by western blot analysis the two main kinase signalling pathways downstream of ASK1. Total MKK3 protein was expressed in thyroid of gsp transgenic mice. However, expression strongly decreased during tumour development (Table 1). The ser189 phospho-MKK3 and ser207 phospho-MKK6 proteins, which are active forms of the kinases, are both recognized indifferently by the antibody we used. Phosphorylated proteins were strongly detected in gsp thyroids independently of the tumour status of the gland (Table 1). Those results demonstrated that in hyperplastic thyroids and adenomas, only MKK6 protein is activated as total MKK3 was poorly expressed. The average expression of total p38-MAPK, the kinase downstream of MKK3/MKK6, was identical in the different groups (Fig. 3 upper panel). By contrast, expression of the p38-phosphorylated active form was strongly decreased during tumour development (Fig. 3 lower panel). Indeed, high level of thr180/tyr182 phospho-p38-MAPK protein was detected in all non pathological thyroids of gsp transgenic mice, whereas the expression of this active form of the protein was low in hyperplastic glands, and very weak or absent in adenomas. Moreover, decrease or absence of p38-MAPK activation correlated with decrease or absence of total MKK3 expression . The other kinases pathway activated by ASK1 implicates SEK1/MKK4 and SAPK/JNK molecules. Total SEK1 protein was detected in thyroids of gsp mice at all stages of tumour development, but level of expression was higher in adenomas than in non pathological and hyperplastic gland. By contrast, the thr261 phospho-SEK1 active form was expressed stronger in non pathological thyroids than in hyperplastic glands or adenomas (Table 1). Although total and thr183/tyr185 phospho-SAPK/JNK proteins level of expression was similar in all groups of mice, apparent molecular weight of phosphorylated proteins was higher in non pathological thyroids compared to disease glands (Table 1). These results suggested that SAPK/JNK proteins are phosphorylated at both thr183 and tyr185 sites in non pathological thyroids, but that hyperplastic glands and adenomas are mono-phosphorylated due to a lack of phospho-SEK1 dependent thr183 phosphorylation. As dual phosphorylation of SAPK/JNK is necessary for kinase activity, these results showed that the ASK1/SEK1/JNK pathway, as well as the ASK1/MKK3/p38-MAPK signalling pathway, are strongly inhibited during tumour development.

### In vitro cultured thyrocytes did not expressed FADD protein.

In the course of our study, we performed *in vitro* cultures of normal non transgenic TFC and investigated the presence of molecules of the Fas signalling pathway by semi-quantitative RT-PCR and western blot analysis. Surprisingly, although FADD mRNA was expressed (Fig. 4A), FADD protein was undetectable in cultured thyrocytes (Fig. 4B). Further investigations showed that loss of FADD protein expression was induced by dissociation of thyroid tissue with collagenase H (Fig. 4B) and that FADD protein could not be detected thereafter, even following 10 days of culture . From the above results, it was important to know whether lack of FADD protein was an intrinsic property of cultured thyroid cell or a generalized FADD protein expression regulation mechanism. Hepatocytes were chosen to compare with thyrocytes because they are epithelial cells and liver tissue, like thyroid tissue, needs collagenase H treatment before culture. Western blot analysis was performed on *ex vivo* liver, collagenase H treated and *in vitro* cultured hepatocytes. *Ex vivo* liver, like *ex vivo* thyroid, expressed FADD protein (Fig. 4C). However, in contrast to TFC, western blotting of collagenase H treated and *in vitro* cultured hepatocytes showed presence of FADD protein (Fig. 4C). These results suggested that loss of FADD protein expression was restricted to thyroid epithelial cells and that this *in vitro* culture system could be used to elucidate the consequences of FADD protein expression regulation by thyrocyte.

### Fas signalling in absence of FADD increases TFC growth.

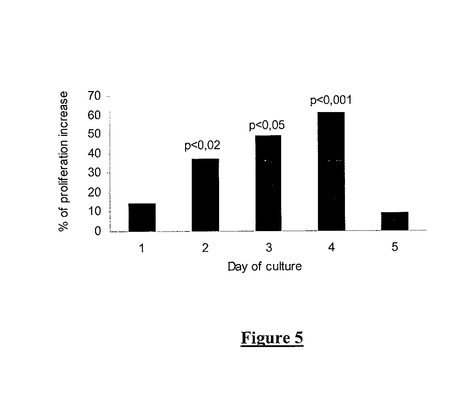

To evaluate the biological consequences of the Fas/FasL interaction in absence of FADD protein, thyroid cells were cultured with or without anti-Fas antibody and proliferation of thyrocytes was determined every day until confluence. Figure 5 showed that after 24h of treatment, there was no significant difference between proliferation of control and anti-Fas treated TFC. Thereafter, anti-Fas antibody accelerated growth of TFC compared to not treated TFC. Moreover, anti-Fas antibody-induced growth increase significantly after 48h, 72h and 96h of treatment (37% (p<0.02), 49% (p<0.05) and 61% (p<0.001) respectively). No more difference between control and anti-Fas treated TFC growth could be detected when cultures reached confluence (after 120h of treatment).

### Discussion

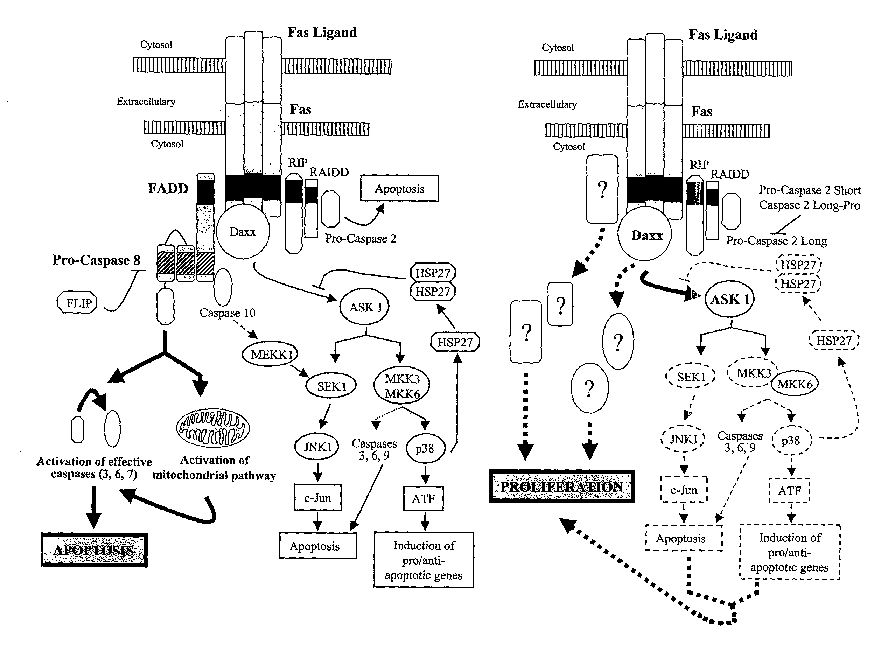

The expression of a large array of molecules involved in the Fas/FasL pathway during the *in vivo* transformation process of murine thyroid follicular cells (TFC) was investigated by using multiple complementary techniques. We showed that during the transformation process from non pathological TFC to hyperplastic nodules and adenoma or adenocarcinoma, 1) FasL expression is gained; 2) FADD protein but not mRNA is lost; 3) Fas signalling in absence of FADD results in a Daxx- but not RIP-mediated signal; 4) disappearance of FADD protein was correlated with inactivated status of the p38-MAPK; 5) Fas signalling in absence of FADD accelerated TFC growth. This is the first instance in which spontaneous FADD protein disappearance has been associated with pathological consequence. In mice, transgenic expression of a mutant form of Gsα subunit directed to TFC induces adenomas or adenocarcinomas. In these mice, pathological lesions start around 8 months of age, and thyroid tumour develops thereafter. To elucidate the pathological event that could explain such tumour development, we focus on the Fas/FasL expression as well as Fas signalling molecules. Indeed, FasL expression could have a double advantage for the cells. First, it was reported that FasL expression on tumour cells permits elimination of infiltrating lymphocytes that express the Fas receptor (counter-attack model). Second, a new concept has recently emerged showing that Fas/FasL interactions not only promote cell death of Fas⁺ target cells, but can also stimulate proliferation of the same Fas⁺ cells. Thus, FasL expression on tumour cells will confer a double advantage to these cells by inducing apoptosis of invading cytotoxic lymphocytes, and by stimulating their own proliferation through Fas/FasL interaction. Nevertheless, regulatory mechanisms that take place in tumour cells to block the death signal, whereas maintaining the transduction of a proliferative signal mediated by Fas, are poorly understand.
The fact that gain of FasL expression during tumour development was concomitant with loss of FADD protein suggested that absence of FADD may allow tumour cells to escape a suicide death that could be induced by Fas/FasL co-expression.
In gsp transgenic mice, our results showed that FADD protein might have a tumour suppressive function. As a matter of fact, Fas signalling in TFC in absence of FADD appears to promote cell growth instead of apoptosis. Indeed, in a culture model of thyrocytes, our results showed that stimulation of TFC by anti-Fas antibody, in absence of FADD, resulted in a higher proliferation of these cells compared to non stimulated cells. Furthermore, those results were confirmed by the higher proliferative rate of TFC expressing FasL, isolated from thyroid specific FasL transgenic mice, compared to non transgenic cultured thyrocytes .
Our results show that gain of FasL, with concomitant loss of FADD expression, could confer multiple advantages to thyroid tumour cells: 1) high resistance to Fas-mediated cell death, 2) induction of proliferation via Fas signalling, 3) potential elimination of infiltrating lymphocytes.
FADD is a common signalling molecule to different death receptors. Stimulation of TNFR1 (tumour necrosis factor (TNF) receptor 1) with TNF leads to recruitment of the adapter TRADD (TNFR-associated death domain) which can in turn bind FADD and TRAF2 (TNFR-associated factor 2). As FADD protein is not expressed in thyroid adenomas and adenocarcinomas, TNF stimulation would result only in a TRAF2 dependent transducing signal. Furthermore, FADD-deficient embryonic fibroblasts were shown to be resistant to apoptosis induced by TNFR1, suggesting that FADD is essential to TNF-mediated cell death. Indeed, in our *in vitro* thyrocytes culture model, we found that FADD deficient TFC were resistant to TNFα-mediated cell death , supporting the idea that gsp transgenic mice acquired Fas- but also TNFR1-induced apoptosis resistance. FADD is also necessary to DR3 (death receptor 3) induced apoptosis, as DR3 resembles TNFR1 and activates similar signalling molecules upon APO3L stimulation. TRAIL (TNF-related apoptosis-inducing ligand) is implicated in tumour cells elimination. In these cells, two death receptors, DR4 (TRAIL-R1) and DR5 (TRAIL-R2), can transduce apoptotic signals. Requirement of FADD for TRAIL-mediated cell death was demonstrated using FADD-deficient embryonic fibroblasts stably transfected with DR4 or DR5). Moreover, primary thyrocytes, as well as thyroid papillary carcinoma-derived cell line KAT5 were shown to express DR4 and DR5 but to resist TRAIL-induced apoptosis. All these data showed that apoptosis induced by Fas, TNFR1, DR3, DR4 and DR5 needs FADD molecule, implicating that all these death pathways may be blocked in gsp transgenic mice developing adenomas or adenocarcinomas. Thus, FADD deficiency would confer multiple resistance against death receptors-induced apoptosis to tumour cells, and would allow opening of multiple proliferation pathways.
Signal transmission through Fas can operate through three different adaptators. In the absence of FADD, only Daxx and RIP proteins can transduce a Fas-mediated signal. However, we have showed here that RIP signalling was not elicited in FADD deficient thyroids, and that ASK1 intermediate expression strongly increased in adenomas/adenocarcinomas, but without consequent p38-MAPK or JNK pathways activation. As Daxx still is a poorly known molecule, we can make three hypothesis concerning the mechanism of Fas-induced proliferation of TFC in absence of FADD: 1) Daxx signalling is directly responsible for cell proliferation, by regulating the balance between MKK3, MKK6 and SEK1/MKK4 activation, 2) Daxx is an adapter protein leading to a still unknown cell growth transducing pathway, 3) Daxx is not implicated in this phenomenon and a still unknown adapter for the Fas receptor transduces a proliferative signal (see scheme on Fig. 6).
Activation of proliferation in response to Fas engagement has been previously described in different cell types. In normal T lymphocytes population, consequence of Fas ligation depends of previous antigenic stimulation. Indeed, following Fas ligation, memory CD4⁺ T cells are induced to proliferate whereas naive CD4⁺ T cells undergo apoptosis both *in vitro* and *in vivo.* In human dermal fibroblasts, induction of apoptosis or proliferation depends from the level of Fas receptor expression. Although anti-Fas antibody stimulates proliferation of low Fas expressing fibroblasts, anti-Fas treatment triggers apoptosis of high Fas expressing fibroblasts that mimic inflamed skin. More surprisingly, in liver, which is a Fas-mediated apoptosis highly sensitive cell, Fas engagement can accelerate organ regeneration after partial hepatectomy in vivo, instead of inducing fulminant hepatitis. All these data demonstrated that signal through Fas can result either in apoptosis or in proliferation, depending of the type of cells and the environmental conditions. In tumour cells, anti-Fas promoted cell growth has been reported in a few cases and concerned hematologic well as non hematologic tumours. In human thyroid carcinoma cells, it was reported that both Fas and FasL were expressed, but that cross-linking of Fas failed to induce recruitment and activation of caspase 8. It would be interesting to test the presence of FADD protein in those cells, as lack of this molecule could explain Fas-mediated cell death resistance, and strong proliferation of tumour cells.
In a more general approach, FADD expression may be tested in all types of cancer expressing Fas and FasL molecules, but resistant to Fas-induced apoptosis.
In eukaryotic cells, translation of mRNAs is mainly due to a cap-dependent mechanism, implicating proteins of the eukaryotic initiation factor 4 (eIF4) family. The multiproteic complex eIF4F can recognize the m⁷GpppN cap structure and initiate the ribosome scanning of the mRNA. On the other hand, few mRNAs are translated by a cap-independent mechanism. In this case, ribosome entry occurs at a specific region in the 5' end of the mRNA called internal ribosome entry segment (IRES). Such mRNAs possessing IRES have been described in mammalians and are implicating in control of cell growth. Among them, c-myc mRNA which can be translated by cap-dependent or cap-independent mechanisms. It was demonstrated that during apoptosis of HeLa cells induced by TRAIL, c-myc mRNA translation occurred via the IRES, and that the p38-MAPK signalling pathway was implicated in this cap-independent translation. Strikingly, the thr180/tyr182 phospho-p38-MAPK pattern of expression was very similar to that of FADD (Fig.2 B and 3). Indeed, although both phospho-p38-MAPK and FADD proteins were expressed in non pathological gsp thyroids, both proteins were absent in adenomas/adenocarcinomas. Importantly, no cap site was found in FADD mRNA. This suggested that, like c-myc, FADD mRNA regulation of cap-independent translation could occur via activation of p38-MAPK pathway. In thyroid tumour cells, inhibition of p38-MAPK signalling would lead to blockade of cap-independent mRNA translation, including FADD and c-myc which are both involved in apoptosis. Absence of expression of these proteins, and particularly FADD, can contribute to cancer progression by deviating FasL signalling from a death to a proliferative pathway. Our data suggest a new role for the Fas/FasL pathway in the etiology of thyroid tumour. In absence of FADD protein, but in presence of Fas and FasL molecules, thyrocytes would have numerous advantages including high resistance to Fas and other death receptors-mediated apoptosis, stimulation of their own proliferation through Fas/FasL interaction, and capacity to counter-attack the infiltrating lymphocytes allowing immune escape.
Understanding how FADD protein expression is lost will permit to envisage new therapeutic approaches. Indeed, restoration of FADD function in FasL⁺ thyrocytes would induce higher Fas-mediated cell death susceptibility, and inhibit Fas-induced proliferation of these cells, leading to tumour regression. Moreover, the present results may reflect a feature which is common to most cancers wherein FasL expression and/or high resistance to apoptosis-inducing drugs have been reported.

### EXAMPLE 2: expression of FADD is also lost in blood samples of myeloid leukaemia human chemo-resistant patients.

Blood samples from human patients for which a diagnosis of acute or chronic myeloid leukaemia (AML or CML) have been established were tested for FADD expression. All blood samples tested contained at least 80% of tumour cells. The results thus obtained are as follows:

**Table 2:**

| **human AML** | | | | |
|---|---|---|---|---|
| Patient's reference name | DISEASE | DIAGNOSIS | Sampling date dd.mm.yy (dose) | **FADD** |
| BE | AML 3 | Cured | 03.04.00 | +++ |
| PA | AML 5 | Cured | 26.09.97 | +++ |
| 3L | AML 5 | 5 then R | 25.09.98 (40.10⁶) | +++ |
| 8R | AML 5 very aggressive | 5 (cured) | 17.10.01 (49.10⁶) | ++ |
| | | | 08.01.02 | + |
| BEL | AML 3 | 5 (cured) | 28.11.01 (7.10⁶) | ++ |
| | | | | + |
| 1A | AML | In remission. Early relapse on March, 02 Death in April 2002 | 10.08.01 (20.10⁶) | +/- |
| PE | AML 3 | In remission. Relapse in Oct 2002 | 20.11.01 (25.10⁶) | +/- |
| 3S | AML 2 | R | 04.04.00 (10.10⁶) | - |
| 4V | AML | R Dead | 27.04.98 (20.10⁶) | - |
| 5A | AML 4 | Highly R Dead | 04.07.01 (15.10⁶) | +/- |
| 2M | AML | R Dead | 27.04.00 (25.10⁶) | +/- |
| CU | AML | R | 19.01.01 (25.10⁶) | + |
| RO | AML | R | 25.01.00 | + |
| ROR | AML | R | 06.03.98 | +/- |
| SA-M | AML | R | 04.04.02 | +/- |
| C*Y* | AML 4 | R | 28.03.97 (50.10⁶) Cellules au diagnostic | - |
| | | Relapse | 20.02.98 | + |
| RAN | AML | | 14.08.02 | + ? |
| BOT | AML | | 20.09.02 | +++ |
| DUP | AML | | 27.09.02 | +++ |
| GOU | AML | | 27.09.02 | - |
| BIL | AML | | 30.09.02 | +++ |
| LET | AML-ALL | | 19.09.02 | + |
| LEB | | | 27.09.02 | - |
| **S** = chemo-sensitive to anti-tumour chemotherapy | | | | |
| **R**= chemo-resistant to anti-tumour chemotherapy | | | | |
| **nd** = not yet determined | | | | |

**Expression of FADD**, as assessed by the intensity of the 28-30 kDa band on Western blots (blind test analysis):
- **- or** +/-: null or very low,
- +: low,
- ++: medium,
- +++: high.

**Table 3:**

| **human CML** | | | | |
|---|---|---|---|---|
| Patient's reference name (or cell line K562) | DISEASE | DIAGNOSIS | Sampling date dd.mm.yy (dose) | FADD |
| K562 | CML line | - | 22.06.01 | ++ |
| K562 | CML line | - | 02.02.01 | +++ |
| K562 | CML line | - | 21.11.01 | - |
| SL | CML | Direct graft, but R. Then IFNα; very sensitive. No relapse for 5 years. | 02.04.96 (25.10⁶) | +++ |
| 1D | CML | S on May 31, 02 then R after 4 months | 31.05.00 (6.10⁶) | +++ |
| HE | CML | IFNα sensitive for several years. Molecular remission. Became R in 2001 | 15.05.98 | - |
| | | | 15.05.98 (30.10⁶) | ? |
| HE | CML | IFNα Resistant Start STI 571 on Nov 14, 01 | 14.11.01 (5.10⁶) Avant STI 571 | -? |
| 1C | CML | R In relapse | 18.05.01 (20.10⁶) | - |
| 2L | CML aggressive | R Relapse after graft, but decrease in bcr-abl mRNA after immunosuppressive treatment has been stopped | 29.03.00 (40.10⁶) | - |
| 30 | CML | R | 05.02.96 | |
| 4E | CML | R | 21.09.98 (30.10⁶) | - |
| 6R | CML | R Relapse after graft | 14.05.98 (17.10⁶) | |
| SB | CML | IFNα- and chemo-Resistant Death during graft | 25.01.96 (20.10⁶) | + |
| FO | CML | R to all STI 571 resistant | 03.06.02 (20.10⁶) | + |
| BR | CML | R | 26.02.96 | - |
| AM | CML | IFNα Resistant Starts STI 571 | 08.01.02 | + |
| RE | CML | R Relapse after graft on Nov 24, 98 | 16.11.98 (20.10⁶) | + |
| RE | CML | Resistant | 05.03.01 | ++ |
| 5R | CML | IFNα Resistant No relapse after graft | 30.03.98 (19.5.10⁶) | -? |
| 5R | CML | Post-graft No relapse | 28.11.01 (5.10⁶) | -? |
| PB | CML | Resistant | 06.05.96 | ? |
| 8A | CML | Grafted in Sept, 00 + immunosuppressive treatment No relapse | 11.05.01 (10.10⁶) graft lymphocytes | - |

**Expression of FADD**, as assessed by the intensity of the 28-30 kDa band on Western blots (blind test analysis):
- **- or** +/-: null or very low,
- +: low,
- ++: medium,
- +++: high.
Anti-tumour treatment = IFNα tentative therapy, followed in case of resistance, by bone marrow allograft (tentative GVL effect via Fas) STI: drug inducing apoptosis by blockade of the bcr-abl pathway

### EXAMPLE 3: FADD protein is released out of normal thyroid lobes (model of tumour development)

Further to the above examples 1 and 2, wherein it is demonstrated that tumoral cells have lost FADD protein expression, experiments were conducted on in vitro cultures of normal thyroid lobes collected from tumour-free mice to search, in this *in vitro* model of FADD protein loss, for the mechanism underlying this post-translational alteration.

### A. Material and methods:

### In vitro culture of normal thyroid lobes:

Normal thyroid lobes of tumour-free mice (CBA/J female mice purchased from Iffa Credo, L'Arbresle, France) were collected and either directly analysed (time 0 *= ex vivo* sample), or incubated in a RPMI medium (RPMI 1640 medium 61870-010, Life Technologies) for various time periods ranging from 2 min to 4h (2 min; 5 min ; 10 min ; 15 min ; 30 min; 45 min; 1h ; 4h).

### Antibodies:

Primary anti-mouse FADD antibodies (goat polyclonal IgG) used were either specific for the N- terminal (N-18, sc-1172, lot H231, Santa Cruz Biotechnology) or C- terminal (M-19 -previously referenced under ref. MC-19-, sc-6036, lot G 199, Santa Cruz Biotechnology). The following control Ab used in this study was a goat polyclonal IgG (Vector, BA-1000 ).
For immunohistochemistry techniques, a biotinylated rabbit anti-goat IgG (Vector, BA-5000) was used as secondary Ab. For Western blot and ELISA techniques, a peroxidase-labeled anti-goat IgG (Ref. A5420 ; Sigma-Aldrich, Saint Quentin Fallavier, France) was used as secondary antibody.

### Immunohistochemistry:

Thyroid lobes were collected and immediately covered in optimal temperature medium (Tissue-Tek; Bayer, Elkhart, IN), slowly frozen by floating in isopentane on liquid nitrogen, and stored at -80°C until cut. Sections of 5-7 µm were cut on a cryostat at -18°C and collected onto SuperFrostplus slides (Roth Sochiel, Lauterbourg, France). Sections were dried overnight and stored at -80°C until use. Before staining, sections were fixed for 10 min in acetone and incubated for 20 min in 0.05 M TBS, pH 7.6, in the presence of 10% normal horse serum, and stained (45 min) with the appropriate primary Ab (10µg/ml). The secondary biotin-conjugated Ab was used at 1µg/ml (30 min). Avidin-alkaline phosphatase (Sigma, St. Louis, MO) third stage (30 min), and FastRed substrate (Acros Organics, Noisy-Le-Grand, France) were used to visualize specific staining. Sections were counterstained in hemalum (Fisher Scientific, Pittsburgh, PA) and mounted in an aqueous mount (Aquatex ; Merck, West Point, PA).

### Western blot:

At the indicated times, supernatants were collected, protein concentration determined using a micro BCA protein assay reagent kit (Pierce, Rockford, Illinois), and stored at -20°C until used. Thyroid lobes were washed in PBS, and total proteins were extracted with lysis buffer (10 mM Tris-HCl, 150 mM NaCl pH 7.8, 1%NP40, 1 mM PMSF, 1 µg/ml aprotinin and 1 µg/ml leupeptin), containing a cocktail of protein phosphatase inhibitors (Calbiochem-Novabiochem Corp., La Jolla, California).
For some experiments, Triton 1% (Sigma-Aldrich, X-114, Saint Quentin Fallavier, France) or Chaps 2% (Sigma-Aldrich, C-9426, Saint Quentin Fallavier, France) was used instead of NP40 1% (Sigma-Aldrich, N-3516, Saint Quentin Fallavier, France).
Sample concentrations were determined using a micro BCA protein assay reagent kit (Pierce, Rockford, Illinois), and either 40 µg or 20 µg of total proteins from thyroid lobes or supernatant culture respectively were subjected to SDS-PAGE and transferred to nitrocellulose (NEN Life Sciences, Boston, Massachusetts). After blotting, membranes were probed with specific primary anti-FADD antibody (M-19, sc-6036, Santa Cruz Biotechnology) was used at 0.2 µg/ml in TBS 0.1% Tween® 20 containing 5% milk overnight at 4°C. The peroxidase-labeled anti-goat IgG secondary antibody was used at a 1:15,000 dilution, and proteins were visualized with the enhanced chemiluminescence technique (Amersham Pharmacia Biotech).

### Detection of FADD in mouse supernatant by ELISA method:

Flat-bottom microtiter plates (Costar, n°3590) were coated overnight with 50 µl of supernatant (5 µg/ml) at 4°C, then washed four times with PBS-Tween 20. Free protein-binding sites were blocked by adding PBS-2% BSA for 2 h at room temperature (RT). After three more washes with PBS-Tween® 20 anti-FADD (M19) antibody (1 µg/ml in PBS-Tween 20-2% BSA) at 4°C, was added for 2 hr at RT. After extensive washing of the plates with PBS-Tween® 20 ; 1:40,000 peroxidase-conjugated anti-goat IgG antibody (Sigma) in PBS-Tween®-BSA was added 1 h at RT, and the colorimetric reaction was revealed by substrate addition (TMB (3,3',5,5'-tetramethyl-benzidine), Sigma ref. T-8665). The plates were read at 450 nm with a Titertek® Multiscan spectrophotometer (Dynatec MR5000, Guyancourt, France).

### B. Results:

### Immunohistochemistry of cultured normal thyroid lobes:

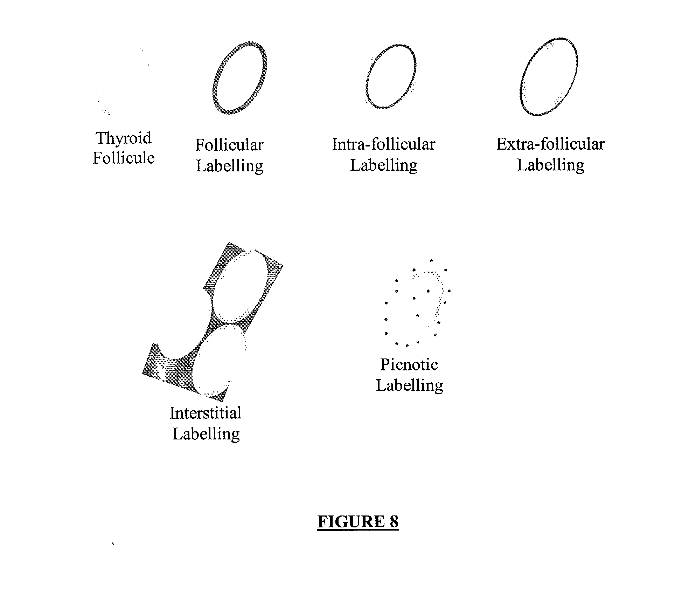

*In vitro* cultured normal thyroid lobes loose FADD expression during *in vitro* culture, and thereby constitute an *in vitro* model of tumour development. These cultures were therefore analysed by immunohistochemistry, and FADD localisation was qualified in accordance with to the schematic definition shown on Figure 8.

### a. C-term targeting:

A first experiment was conducted with the anti-FADD M-19 (goat polyclonal antibody) as a primary antibody. This Ab is directed to the C-terminal part of the FADD protein.

### Ex vivo thyroid (time 0):

At a concentration of 10 µg/mL in M-19 Ab, a picnotic and heavy follicular labelling surrounds the nucleus. By « picnotic » labelling, it is herein meant a granular labelling, which evokes a vesicular localization of the targeted protein.

### Thyroid at 2 min:

A picnotic and heavy follicular labelling surrounds the nucleus.

### Thyroid at 5 min:

The labelling is lighter than the one which was observed on the *ex vivo* and 2 min thyroid ; it looks less follicular and more interstitial.

### Thyroid at 10 min:

The labelling becomes lighter around the nuclei, and is not picnotic anymore. It tends to be present in the colloid or in the interstitial space.

### Thyroid at 15 min:

The labelling is very light. The picnotic labelling has disappeared from around the nuclei, and the colloid that is now labelled.

### Thyroid at 30 min:

The cultured cells seem to have come back to the *ex vivo* stage, with a picnotic labelling which surrounds the nuclei, but which is somehow more spread out, as in the present "thyroid at 30 min" case, the interstitial space and the colloid are labelled as well.

### Thyroid at 1h:

Labelling is lighter than at 30min. The picnotic labelling looks weak around the nuclei, but is found at the colloid or interstitial level again. This is supportive of a mechanism by which the cultured cells would re-start to excrete FADD proteins.

### b. N-term targeting:

A second experience has been conducted with the anti-FADD N-18, which is an Ab directed to the N-terminal part of the FADD protein.

### Ex vivo thyroid (time 0):

At a concentration of 10 µg/mL of N-18 Ab, the labelling looks follicular, and picnotic. Labelling intensity is higher than the one which was observed with the C-term targeted M-19 Ab.

### Thyroid at 5 min :

Labelling is a lot lighter than the one which was observed on the *ex vivo* thyroid. This supports the view that the rate at which the FADD protein disappears is very fast. The labelling is diffuse, not picnotic, and is located in the interstitial space.

### Thyroid at 10 min :

The labelling is very light, extra-follicular and interstitial ; it is not picnotic, and there is no labelling in the colloid any more.

### Thyroid at 15min :

The follicular (non picnotic) labelling seems heavier than at 10min, but overall labelling remains quite light.

### Thyroid at 30min :

Some picnotic labelling surrounds the follicules. The labelling is extra-follicular, and is lighter than at the *ex vivo* stage.

### Thyroid at 1 h:

The thyroid is not labelled any more.

### c. Both C-term and N-term targetings:

A third labelling experiment has been conducted with a mixture of both the C-term-targeted Ab M-19 and the N-term-targeted Ab N-18 (10 µg/mL of each antibody).

### Ex vivo thyroid (time 0):

At a concentration of 10µg/mL of each antibody, the labelling intensity is very strong. Labelling is follicular, and looks very picnotic.

### Thyroid at 5min:

The labelling is lighter than at the ex vivo stage, but is still follicular and picnotic. Labelling is not look interstitial, but rather looks intra-follicular for some follicules.

### Thyroid at 10min:

The labelling is lighter than at the 5min stage, and is mainly follicular. There is practically no picnotic labelling any more.

### Thyroid at 15min:

Labelling is heavier than at the 10min stage, but is clearly lighter than at the *ex vivo* stage. Overall labelling is follicular, and some follicules are intra-follicularly labelled. Some picnotic labelling is observed, but only in small amounts.

### Thyroid at 30min:

The labelling intensity is similar to the one observed at the 15min stage, or slightly lighter. Labelling is mainly extra-follicular, and weaker than at the *ex vivo* stage. There is some picnotic labelling.

### Thyroid at 1h :

The labelling intensity is very weak compared to the ones observed at the *ex vivo* and the 30min stages. Certain thyroid follicules are practically unlabelled. There is a lot of intra-colloidal picnotic labelling.

### Results of Western blot technique on cultured normal thyroid lobes:

Western blots of the proteins extracted from the cultured thyroid lobes confirm that these thyroid cells lose FADD protein expression when cultured *in vitro.* This protein loss is very fast, as it is already observed after 5-10min of culture. Extraction of the cell proteins with a lysis buffer containing Triton or Chaps instead of NP-40 gave similar results, thereby confirming that the absence of FADD in the protein extracts is not due to a problem of protein solubilization. Incubation of the thyroid cultures at 4°C, or incubation of the thyroid lobes for 1 or 2h in a RPMI medium wherein brefeldin A had been added did not block the loss of FADD protein expression.

### Results of Western blot and of ELISA techniques on culture supernatants:

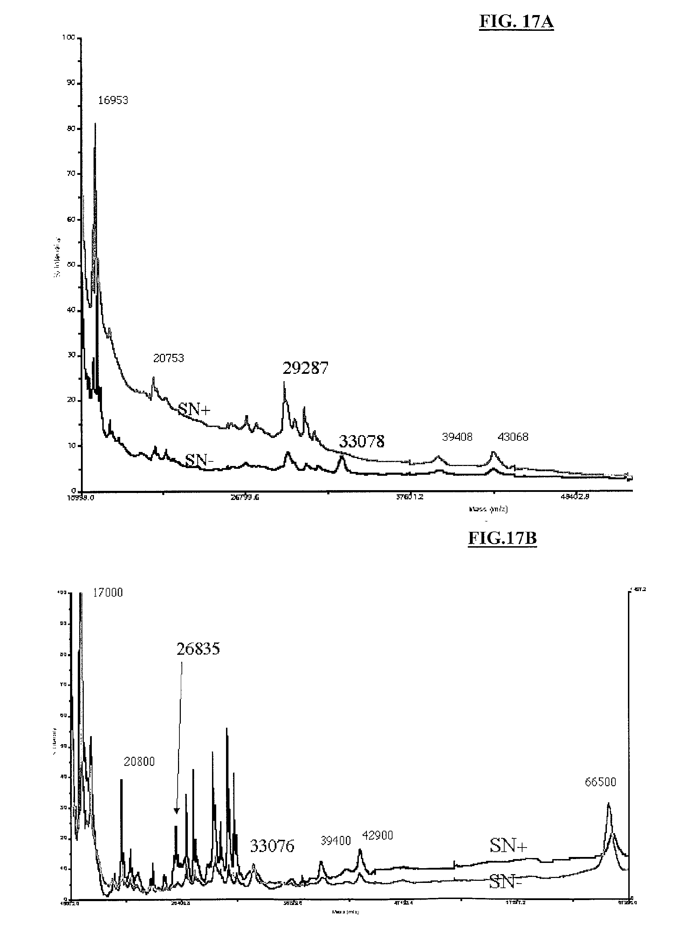

Western blots and ELISA analyses of culture supernatants indicate that FADD protein is present in the culture supernatants as soon as 2min after starting *in vitro* culture. FADD protein is also detected in the supernatants at 5min, 10min, 15min, 30min, 45min, 1h, and 4h of *in vitro* culture.
In these culture supernatants, FADD protein is detected at a MW of about 30-32kDa on Western blots.
Moreover, mass spectrometry analyses of culture supernatant showed a protein peak at 33kDa. See Figures 17A and 17B, which show mass spectrometry analysis of supernatants of 1h-old culture of normal thyroid lobes (SN+: in the presence of 2-mercaptoethanol ; SN-: in the absence of 2-mercaptoethanol). Figure 17A shows a representative direct analysis of a 1h culture supernatant (% of peak intensity as a function of MW in Da), and Figure 17B shows a similar analysis performed on a supernatant that has been treated on a C4 column to concentrate and eliminate the salts thereof. In the absence of 2-mercaptoethanol, a protein of about 33kDa is detected in the supernatant, whereas in the presence of 2-mercaptoethanol, the protein has a MW of about 28kDa (Figure 17A). When treated both by 2-mercaptoethanol, and treatment on C4 column so as to concentrate and eliminate the salts from the supernatant, the number of peaks between 26kDa and 30kDa increases.

### C. Discussion :

Immunohistochemistry analyses over time gave similar results, whether an antibody directed to FADD C-terminal part, or an antibody directed to FADD N-terminal part, or a mixture of both antibodies is used.
These immunohistochemistry analyses confirm that FADD protein expression is lost *in vitro* culture of normal thyroid cells. Loss is observed as soon as after 5-10min of culture.
Analysis of the culture supernatants indicate that FADD protein is lost by a mechanism of excretion. All or some FADD proteins might be secreted in the colloid of the thyroid follicules, and then excreted by transcytosis.
The results also strongly suggest that the localization of the FADD protein may be characteristic of the cell type and/or of the physiopathological status.
As FADD protein is detected at a MW of 30-32kDa in the culture supernatants, whereas its normal MW is of around 28kDa, FADD may be complexed or somehow linked to other molecules during the course of or after its excretion. The mass spectrometro results illustrated by Figures 17A and 17B suggest that *disulfide* bonds may link FADD to another or other entity(ies).

### EXAMPLE 4: FADD protein is present in the sera of adenoma-adenocarcinoma diseased gsp transgenic mice, and is detectable in the serum with a 35 kDa MW

### A. Material and Methods:

### Mice:

Gsp transgenic mice were obtained as described in Michiels, 1994 (PNAS Vol.91 pp.10488-10492). They have no abnormality up to 8 months of age, and thereafter develop hyperplasia followed by hyperfunctioning thyroid adenomas or adenocarcinomas.
Tumour-free mice were used as controls (CBA/J female mice purchased from Iffa Credo, L'Arbresle, France).

### Detection of FADD proteins in the sera of gsp transgenic mice by Western blot:

At different stages of the disease, the sera of the mice were collected, protein concentration determined using a micro BCA protein assay reagent kit (Pierce, Rockford, Illinois), and stored at -20°C until used. 40 µg of total proteins from sera were subjected to SDS-PAGE and transferred to nitrocellulose (NEN Life Sciences, Boston, Massachusetts). After blotting, membranes were probed with specific primary anti-FADD antibody (M-19, sc-6036, Santa Cruz Biotechnology) which was used at 0.2 µg/ml in TBS 0.1% Tween® 20 containing 5% milk overnight at 4°C. The secondary antibody was peroxidase-labeled anti-goat IgG (Sigma-aldrich, Saint Quentin Fallavier, France, ref. A5420) (1:15,000 dilution). To avoid any cross-reacting activity with normal mouse Ig, the secondary Ab was pre-incubated for 30min at 4°C with 1% normal mouse serum. The proteins were visualized with the enhanced chemiluminescencc technique (Amersham Pharmacia Biotech).

### Vital coloration - The release of FADD proteins is an active process:

Thyroid lobes were incubated for 1 hour in RPMI (ref. 61870-010 RPMI 1640 medium, Life Technologies), and then 5 min with a vital colorant (blue trypan), and then immediately covered in optimal temperature medium (Tissue-Tek; Bayer, Elkhart, IN), slowly frozen by floating in isopentane on liquid nitrogen, and stored at -80°C until cut. Sections of 6, 10 and 20 um were cut on a cryostat at -18°C and collected onto SuperFrostplus slides (Roth Sochiel, Lauterbourg, France). Sections were dried overnight and stored at -80°C until use. The sections were then observed under microscope.

### B. Results :

FADD protein was detected in the sera of gsp transgenic mice, at the different stages of tumour development: all mice at the adenoma-adenocarcinoma stage (4 over 4) were serum-positive for FADD proteins in amounts ranging from high to low, and 1 hyperplasic mice over 3 was highly serum-positive for FADD protein (the two other hyperplasic mice were serum-negative for FADD protein).
FADD protein has never been detected in the sera of control tumour-free mice (4 mice over 4).

In the sera of gsp transgenic mice, FADD protein is detected with a MW of 35kDa, whereas its normal MW is of about 28kDa. As indicated in the above example 3, FADD protein was detected at a MW of about 30-32kDa in the supernatants of *in vitro* culture of normal thyrocytes *(in vitro* model of FADD regulation, mimicking FADD regulation during tumour development). The option that FADD protein might be complexed or somehow linked to another or other molecules when (or after) its excretion is thus consistent with the observations made on an in vivo non-human model.

Thyroid cells of gsp transgenic mice which had lost FADD protein expression were not stained after incubation with blue trypan. Therefore, these cells were living cells, and their plasmatic membranes were not permeable. This demonstrates that FADD protein excretion is an active process, and is not due to a simple permeabilization of the thyroid cell membranes.

### EXAMPLE 5:

- FADD protein is detected in the serum of CML and AML human patients
- a low level of FADD protein expression in CML or AML cells is correlated with resistance of the patient to chemotherapy
- a high level of FADD protein in the serum of CML or AML patients is predictive of resistance of these patients to chemotherapy

### A. Material and methods:

### Biological samples:

Blood samples were collected from patients at diagnosis before treatment.
THP1, K562 (CCL-243), and Jurkat (clone E6.1) cell lines are available from ATCC (THP1 accession number = TIB202 ; K652 accession number = CCL-243 ; Jurkat accession number = TIB152).

### Western blot:

Isolated leukemic cells were washed in PBS, and total proteins were extracted with lysis buffer (10 mM Tris-HCl, 150 mM NaCl pH 7.8, 1%NP40, 1 mM PMSF, 1 µg/ml aprotinin and 1 µg/ml leupeptin), containing a cocktail of protein phosphatase inhibitors (Calbiochem-Novabiochem Corp., La Jolla, California). Sample concentrations were determined using a micro BCA protein assay reagent kit (Pierce, Rockford, Illinois), and 30 µg of total proteins were subjected to SDS-PAGE and transferred to nitrocellulose (NEN Life Sciences, Boston, Massachusetts). After blotting, membranes were probed with specific primary antibodies overnight at 4°C: anti-FADD (125-140, Calbiochem, Calbiochem-Novabiochem Corp.) was used at 1 µg/ml in TBS 0.1% Tween® 20 containing 5% BSA. The secondary antibodies were peroxidase-labeled anti-rabbit IgG (Amersham Pharmacia Biotech, Orsay, France, 1:5,000 dilution). The proteins were visualized with the enhanced chemiluminescence technique (Amersham Pharmacia Biotech).

### ELISA method:

96-weel microtiter plates (Costar, n°3590) were coated with rabbit anti-human FADD Ig (125-140, Calbiochem ref. 341282) at Ci: 1mg/ml, Cf: 5µg/ml en PBS, with a Vf of 50µl/weel, incubated overnight at 4°C, and then washed four times (PBS 0.05% Tween® 20). Free protein-binding sites were blocked by adding PBS-2% BSA for 2h at room temperature (Vf: 200 µl/weel). After two more washes with PBS 0.05% Tween® 20, plates were coated with human sera (undiluted, and then sequentially 1:2 diluted with PBS 2% BSA 0.05% Tween® 20) at a Vf of 50µl/weel, and incubated for 2h at room temperature. After four more washes (PBS 0.05% Tween® 20), plates were coated with a secondary antibody (mouse anti-human FADD IgG1 ; clone A66-2, BD Pharmingen ref. 556402 -previously referenced under 65751A) at a Vf of 50 µl/weel, and then incubated for 1h at room temperature. After five more washes (PBS 0.05% Tween® 20), plates were coated with the peroxidase-conjugated antibody (anti-mouse IgG1 PO Caltag ref. 32107), at a Cf of 1-8,000 in PBS 2% BSA 0.05% Tween® 20 in a Vf of 5µl/weel, and were incubated for 1h at room temperature. After five washes in PBS 0.05% Tween® 20, the colorometric reaction was revealed after 15min to 2h of incubation with enzyme substrate (TMB (3,3',5,5'-tetramethyl-benzidine), Sigma ref. T-8665, at a Vf of 50 µl/weel) at room temperature (or 37°C). The colorimetric reaction is stopped by addition of H₂SO₄ 2N at 25 µl/weel. The plates were read at 450 nm (reference wave length = 630nm) on a Titertek® Multiscan spectrophotometer (Dynatec MR5000, Guyancourt, France).

### B. Results:

### Results on high/low level of FADD protein expression in CML, AML and ALL cells and resistance to chemotherapy:

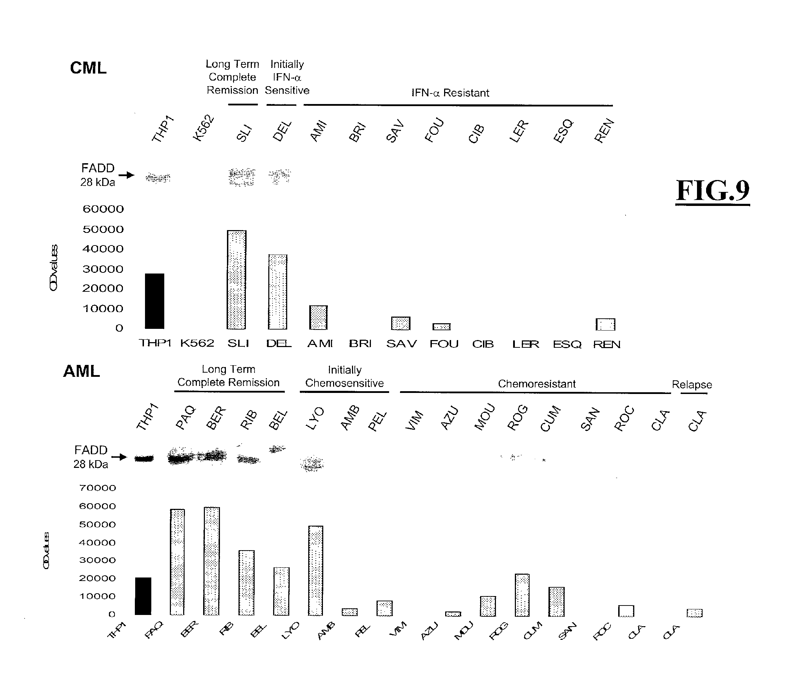

FADD protein expression has been analyzed in leukemic cells of 28 patients at diagnosis (10 CML, 15 de novo AML and 4 ALL). All patients were studied before the initiation of a treatment (IFN-α for CML or standard induction chemotherapy followed by consolidation with or without autologous stem cell transplantation for AML patients).
Western blot analysis of their cells was performed to detect whether FADD presence or absence could be correlated with their pathological status. Representative results of such Western blot analyses are shown on Figure 9 (CML= chronic myeloid leukaemia ; AML = acute myeloid leukaemia ; ALL = acute lymphoid leukaemia ; THP1 = myeloid cell line ; K562 = CML cell line ; Jurkat = ALL cell line, other codes on the top of each lane are representative of individual patients).
Western blot analysis of CML samples showed that FADD was expressed in two IFN-α sensitive patients whereas its expression was very low (4/8) or absent (4/8)
in patients resistant to IFN-α.
Similarly, FADD was highly expressed in leukemic cells of chemosensitive AML patients (5/7). Four out of 7 are in long term complete remission.
Moreover, its expression was very low (4/8) or absent (4/8) in chemoresistant patients.

The intensity of Western blot bands (shown on Figure 9) has been quantified and expressed in OD values. Results are as follows:

**Table 4:**

| **OD values for cellular FADD of CML patients** **(except THP 1 and K652 which are reference cell lines)** | |
|---|---|
| OD values | |
| THP1 | 27751 |
| K562 | 0 |
| SLI | 49645 |
| DEL | 37860 |
| AMI | 12221 |
| BRI | 0 |
| SAV | 6647 |
| FOU | 3329 |
| CIB | 0 |
| LER | 0 |
| ESQ | 0 |
| REN | 6003 |

**Table 5:**

| **OD values for cellular FADD of AML patients** **(except THP 1 which is a reference cell line)** | |
|---|---|
| OD values | |
| THP1 | 21014 |
| PAQ | 58775 |
| BER | 59748 |
| RIB | 36395 |
| BEL | 26864 |
| LYO | 49844 |
| AMB | 4203 |
| PEL | 8351 |
| VIM | 0 |
| AZU | 2351 |
| MOU | 11214 |
| ROG | 23814 |
| CUM | 16507 |
| SAN | 0 |
| ROC | 6593 |
| CLA | 0 |
| CLA | 4285 |

On the contrary, FADD protein was not detected in chemosensitive ALL samples or was strongly expressed in 2/3 chemoresistant ALL.
In summary, we observed that the absence of FADD protein expression in myeloid leukemic cells is correlated to resistance to IFN-α or chemotherapy (p=0.0002).

### FADD protein is detected in the serum of CML and AML patients, and a high FADD level in serum is correlated with resistance to IFNα or chemotherapy :

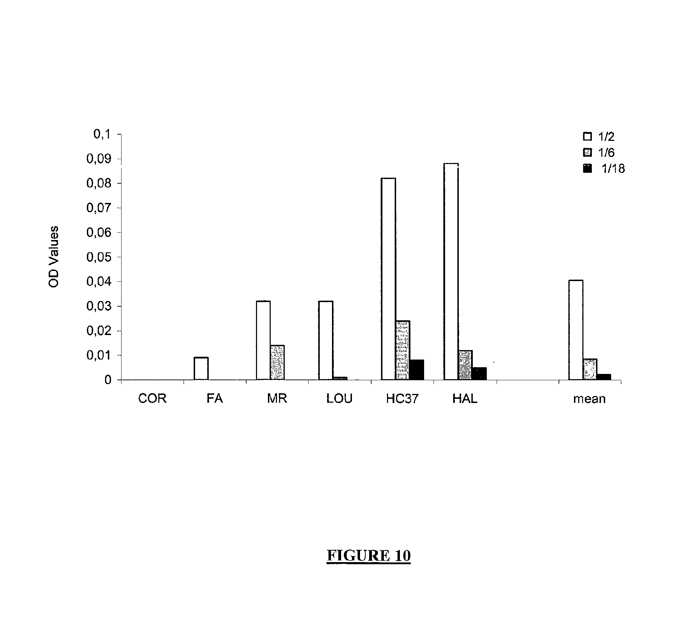

As do the leukemic cells, the sera samples date from the day of diagnosis, *i.e.* before any treatment had started. FADD detection in serum was made by ELISA assays.
Representative results are shown in the below tables below, and on the corresponding Figures 10 to 14, where measured OD values are shown for each patient, at various dilution rates.
In connection with the reactions to tentative treatments, "R" means that the patient was resistant to therapy treatment, "S" that he was sensitive to therapy treatment.

**Table 6:**

| **OD values for healthy patients (control):** | | | |
|---|---|---|---|
| Patient code | ½ dilution | 1/6 dilution | 1/18 dilution |
| COR | 0 | 0 | 0 |
| FA | 0.009 | 0 | 0 |
| MR | 0.032 | 0.014 | 0 |
| LOU | 0.032 | 0.001 | 0 |
| HC37 | 0.082 | 0.024 | 0.008 |
| HAL | 0.088 | 0.012 | 0.005 |
| | | | |
| *mean* | ***0.041*** | ***0.009*** | ***0.002*** |
| *SEM* | ***0.015*** | ***0.004*** | ***0.001*** |

A graphic representation of these results is shown on Figure 10.

**Table 7:**

| **OD values for CML patients:** | | | | |
|---|---|---|---|---|
| Patient code | Reaction to treatment | Undiluted | 1/10 dilution | 1/100 dilution |
| | | | | |
| Healthy | - | 0.006 | 0 | 0 |
| DEL | S, then R after 4 months of treatment | 0.048 | 0.014 | 0.025 |
| HEN | S, then R to IFNα after 3 years of treatment | 0.009 | 0 | 0 |
| LER | R, relapse after graft, but decrease in bcr-abl mRNAs after immunosuppressive treatment has been stopped | 0.079 | 0 | 0 |

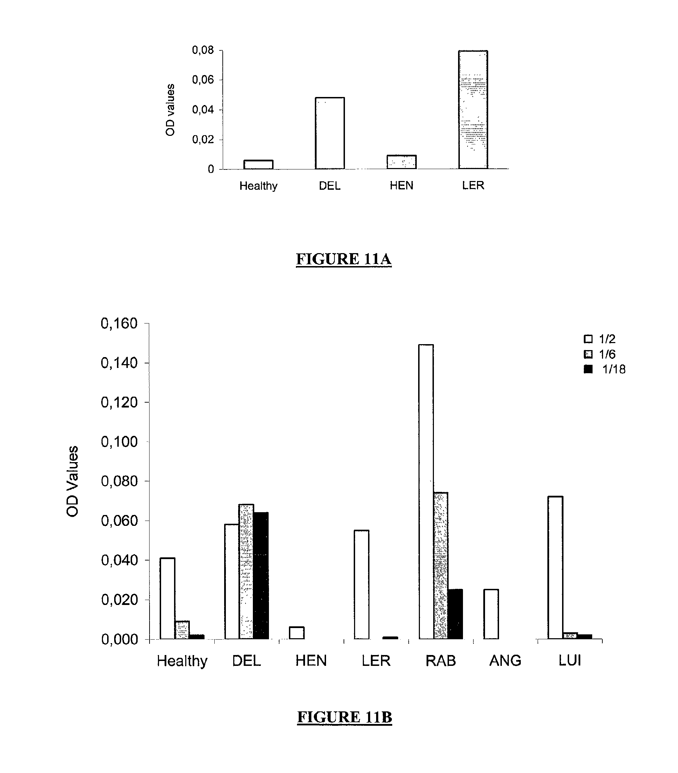

A graphic representation of these results is shown on Figure 11A (undiluted samples).

**Table 8:**

| **OD values of CML patients (other sample dilutions):** | | | | |
|---|---|---|---|---|
| Patient code | Reaction to treatment | ½ dilution | 1/6 dilution | 1/18 dilution |
| | | | | |
| Healthy (n=6) | - | 0.041 | 0.009 | 0.002 |
| DEL | S, then R after 4 months of treatment | 0.058 | 0.068 | 0.064 |
| HEN | S, then R to IFNα after 3 years of treatment | 0.006 | 0 | 0 |
| LER | R, relapse after graft, but decrease in ber-abl mRNAs after immunosuppressive treatment has been stopped | 0.055 | 0 | 0.001 |
| RAB | R, and relapse after graft | 0.149 | 0.074 | 0.025 |
| ANG | graft + immunosuppressive treatment , no relapse (transplant lymphocytes) | 0.025 | 0 | 0 |
| LUI | R | 0.072 | 0.003 | 0.002 |

A graphic representation of these values is shown on Figure 11B.

**Table 9:**

| **OD values for AML patients:** | | | | |
|---|---|---|---|---|
| Patient code | Reaction to treatment | Undiluted | 1/10 dilution | 1/100 dilution |
| | | | | |
| Healthy | - | 0.006 | 0 | 0 |
| PAQ | cured | 0.036 | 0 | 0 |
| BER | cured | 0.066 | 0.004 | 0 |
| LYO | S, then R | 0.097 | 0.016 | 0 |
| VIM | R (dead) | 0.057 | 0.007 | 0 |
| AZU | highly R (dead) | 0.165 | 0.024 | 0.01 |
| MOU | R (dead) | 0.069 | 0 | 0 |
| ROC | R | 0.306 | 0.077 | 0.01 |
| CLA | R, then relapse | 0.302 | 0.034 | 0 |

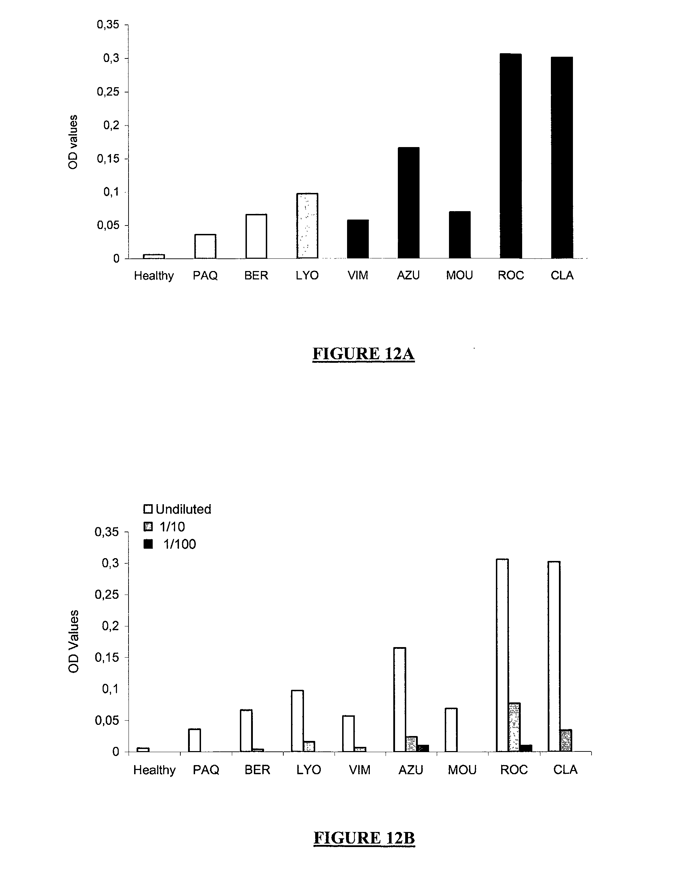

A graphic representation of these results is shown on Figure 12A (undiluted serum samples), and on Figure 12B (undiluted ; 1/10 diluted ; 1/100 diluted)

**Table 10:**

| **OD values for AML patients (other sample dilutions):** | | | | |
|---|---|---|---|---|
| Patient code | Reaction to treatment | ½ dilution | 1/6 dilution | 1/18 dilution |
| | | | | |
| Healthy | - | 0.041 | 0.009 | 0.002 |
| PAQ | cured | 0.021 | 0 | 0 |
| BER | cured | 0.042 | 0.01 | 0 |
| LYO | S, then R | 0.112 | 0.042 | 0.018 |
| ROC | R | 0.383 | 0.179 | 0.052 |
| CLA | R, then relapse | 0.296 | 0.103 | 0.047 |
| ROG | R to all treatments | 0.018 | 0 | 0 |
| POC | R | 0.078 | 0.024 | 0.002 |
| SEK | R | 0.044 | 0.006 | 0 |
| PRO | R | 0.096 | 0.032 | 0.009 |
| OHA | R | 0.008 | 0 | 0 |

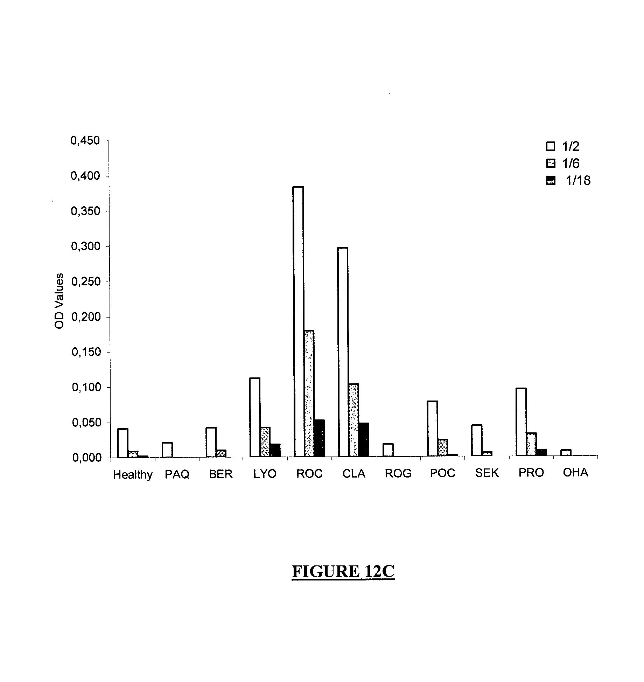

A graphic representation of these results is shown on Figure 12C.

**Table 11:**

| **OD values for ALL patients:** | | | | |
|---|---|---|---|---|
| | Reaction to treatment | ½ dilution | 1/6 dilution | 1/18 dilution |
| | | | | |
| Healthy | - | 0.041 | 0.009 | 0.002 |
| MER | S | 0.007 | 0 | 0 |
| NGU (BUT Western blot analysis of cells shows FADD positive) | R | 0.001 | 0 | 0 |
| BED (Western blots analysis of cells shows FADD negative) | R | 0.044 | 0.008 | 0.013 |

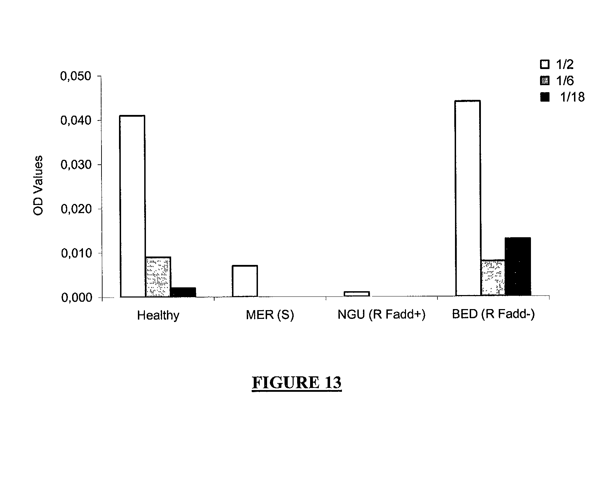

A graphic representation of these results is shown on Figure 13.

**Table 12:**

| **comparative table of measured OD values:** | |
|---|---|
| | 1/2 dilution |
| **HEALTHY patients** | |
| COR | 0 |
| FA | 0.009 |
| MR | 0.032 |
| LOU | 0.032 |
| HC37 | 0.082 |
| HAL | 0.088 |

| **CML patients** | |
|---|---|
| DEL | 0.058 |
| HEN | 0.006 |
| LER | 0.055 |
| RAB | 0.149 |
| ANG | 0.025 |
| LUI | 0.072 |

| **AML patients** | |
|---|---|
| PAQ | 0.021 |
| BER | 0.042 |
| LYO | 0.112 |
| ROC | 0.383 |
| CLA | 0.296 |
| ROG | 0.018 |
| POC | 0.078 |
| SEK | 0.044 |
| PRO | 0.096 |
| OHA | 0.008 |

| **ALL patients** | |
|---|---|
| MER (S) | 0.007 |
| NGU (R Fadd+) | 0.001 |
| BED (R Fadd-) | 0.044 |

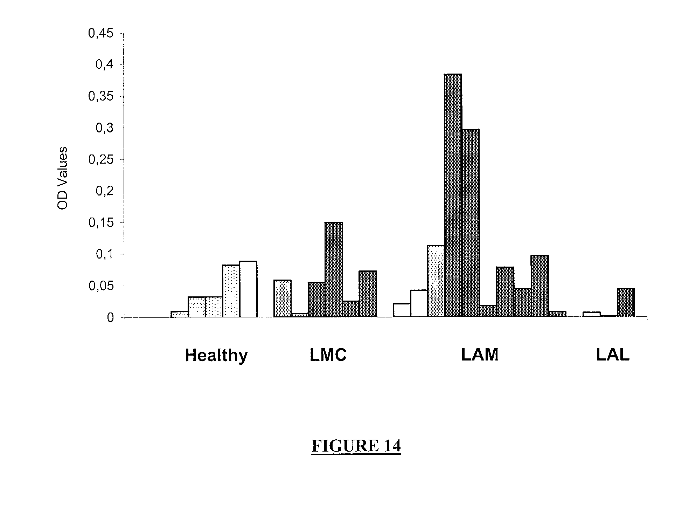

A graphic representation of these results is shown on Figure 14.

FADD protein is detected in the serum of CML and AML patients, and also in some healthy donors at low levels. Presently we don't know if the presence of low amount of FADD in the sera of healthy patients is a physiological phenomenon or an artefact.

Among the CML patients, the amount of FADD protein in the serum is higher in the patient who was later on shown to be resistant to alpha interferon treatment
(LER) than in those patients who were IFNα-sensitive (DEL and HEN).
Quite similarly, there is only little FADD protein in the serum of those patients who will later on reach a complete remission stage from AML (patients PAQ and BER), and in the serum of the patient who was later on shown to be sensitive to chemotherapy (LYO). Three resistant patients over five (AZU, ROC and CLA) have very high levels of FADD protein in their serum, while the serous FADD levels of the two other patients (VIM and MOU) are quite similar to those measured in those patients who were sensitive to treatment.
These results confirm that when a CML or an AML develops, FADD proteins are excreted in the extracellular environment (in the serum of patients).
A high amount of FADD protein in the serum is closely correlated to resistance to tentative therapy (alpha interferon treatment for CML and standard induction chemotherapy for AML). Detecting and measuring the amount of FADD protein in the serum is therefore a reliable tool for predicting resistance/sensitivity to CML/AML standard treatment.
But, when FADD protein is detected in only low amounts in the serum, it is highly recommended to assess FADD expression by the cells to reach a clear prognostic conclusion : level of FADD expression by the cells is indeed a reliable bijective marker, as low cell FADD expression is correlated to resistance to alpha interferon therapy or chemotherapy, and conversely, high cell FADD expression is correlated to sensitivity to alpha interferon treatment or chemotherapy.
The preliminary data thus far obtained for ALL do not lead to similar conclusion on the prognosis value of the FADD test as serous FADD levels of sensitive and resistant patients are very low or similar to those measured in healthy donors sera. However, the serous FADD levels were inversely correlated to the cellular FADD protein levels (NGU: cells +++, serum -; BED: cells -, serum +) suggesting that when FADD is absent in the cells it is excreted in the serum. The physiological relevance of this inverse correlation needs to be clarified.

### EXAMPLE 6: Tentative restoration of FADD protein expression

### A.Material and methods:

Thyroid lobes were collected from normal CBA/J female mice (Iffa Credo, L'Arbresle, France) after sacrifice and intra-cardiac puncture, and incubated in RPMI medium (RPMI 1640 medium, ref. 61870-010, Life Technologies) at 37 °C in a 5% CO₂ atmosphere for different period of time *(ex vivo* thyroid time 0, thyroid 2min, thyroid 5min, thyroid 10min thyroid 15min, thyroid 30min, thyroid 45min, thyroid 1h, thyroid 2h, thyroid 4h).
On the other hand, they were subjected to various treatments for 1h and/or 2h at 37°C in a 5% CO₂ atmosphere.
Treatments were as follows:
- incubation at 4°C,
- incubation in the presence of cytochalasine (inhibits those cellular movements which are associated with actin filaments),
- incubation in the presence of brefeldine A (inhibits those cellular movements which are associated with the Golgi apparatus),
- incubation in the presence of orthophenantroline (inhibits ADAM proteases),
- incubation in the presence of alcohol (which is a solvent for certain of the tested inhibitors).
Lobes and culture supernatant were collected at the end of treatment, lobes were lysed, and FADD protein was thereafter assayed for in the thyroid lobes and in the culture supernatant by Western blot analysis, as described in the above example 3.

### B. Results :

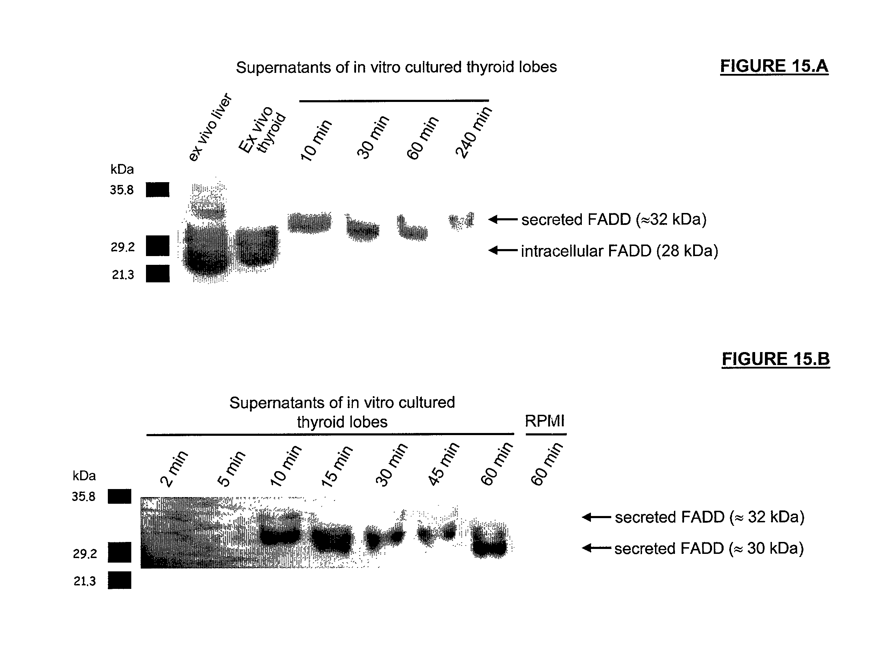

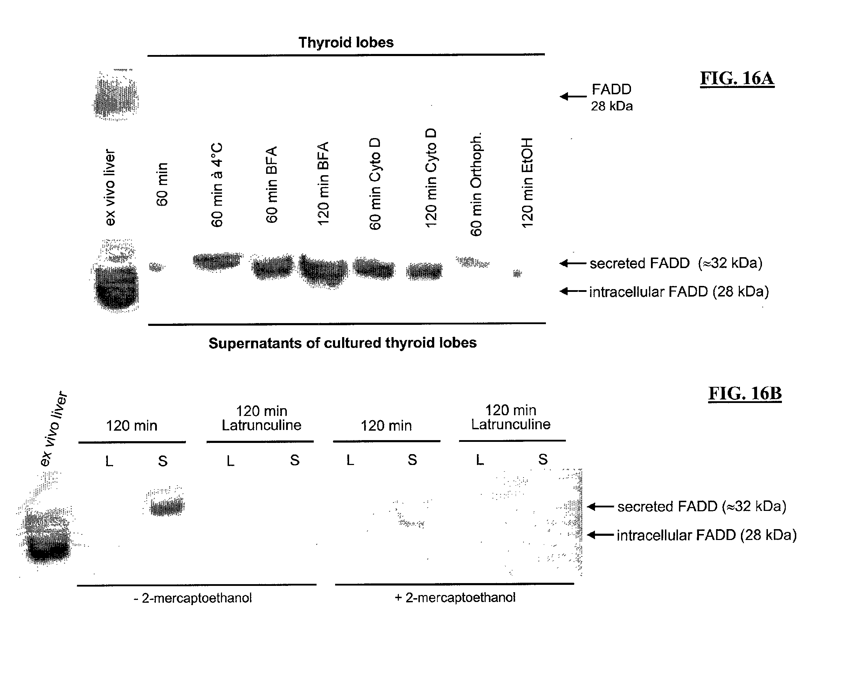

Results are shown on Figures 15A, 15B and 16A, 16B.
Figures 15A and 15B show the kinetic of FADD release from cells of *in vitro* cultured thyroid lobes without any tentative treatment (Western blot).
Figures 16A and 16B show Western blot analysis of thyroid lobes and corresponding culture supernatants, after tentative therapy : the nature of the assayed treatment is indicated next to each lane on top of the blot. L means lobes ; S means supernatant.
None of the treatment that were assayed enabled to stop FADD proteins form being excreted in the extracellular environment. Moreover, samples treatment with 2-mercaptoethanol results in a decreased of FADD apparent MW to 28-30kDa (Fig. 16B). This result was confirmed by mass spectrometry (see Figures 17A and 17B, *ref. supra*). These data confirmed that FADD protein may be linked to other molecule(s) by di-sulfide bridges during the course of or after its excretion.

## Claims

1. *In vitro* use of at least one protein selected from the group consisting of FADD proteins and phosphorylated p38-MAPKs, as an indicator of tumour status.

2. The *in vitro* use of claim 1, **characterized in that** said at least one protein is used as indicator of a status of tumour presence/absence or tumour development/regression.

3. The *in vitro* use according to claim 2, **characterized in that** the cellular amount of said at least one protein is used as a cell indicator of presence/absence or development/regression of a tumour.

4. The *in vitro* use according to claim 2, **characterized in that** the extracellular amount of FADD proteins is used as an extracellular indicator of presence/absence or development/regression of a tumour.

5. The *in vitro* use according to any one of claims 2-4, **characterized in that** said tumour is an adenoma or an adenocarcinoma, such as a thyroid adenoma or a thyroid adenocarcinoma.

6. The *in vitro* use according to claim 1, **characterized in that** a FADD protein is used as an indicator of status of tumour chemo-resistance.

7. The *in vitro* use according to claim 6, **characterized in that** said FADD protein is used as predictive of resistance to an anti-myeloid tumour chemotherapy.

8. The *in vitro* use according to claim 6 or 7, **characterized in that** the cellular amount of FADD proteins which is present in cells which are representative of said tumour, and/or the extracellular amount of FADD proteins which is present in the extracellular environment of such cells is used as predictive of said chemoresistance.

9. The *in vitro* use according to any one of claims 6-8, **characterized in that** said anti-tumour chemotherapy is selected from the group consisting of an interferon treatment such as an alpha interferon treatment, an anthracycline treatment such as doxorubicin, a standard or high dose cytarabin treatment and a vepeside treatment, cytostatic used alone or in combination.

10. The *in vitro* use according to any one of claims 6-9, **characterized in that** said tumour is myeloid tumour, such as a CML or an AML.

11. An *in vitro* method to determine a status of tumour absence/presence or regression/development, **characterized in that** said status is determined:
- by evaluating whether the cells of said tumour or suspected tumour have, when compared to non-tumour but otherwise equivalent cells, lost an abnormal amount of FADD proteins, and/or when they have extracellularly released an abnormal amount of FADD proteins, and/or
- by evaluating whether the cells of said tumour or suspected tumour have, when compared to non-tumour but otherwise equivalent cells, an abnormally low amount of phosphorylated p38-MAPKs.

12. The *in vitro* method of claim 11, **characterized in that** said tumour is an adenoma or adenocarcinoma, such as a thyroid adenoma or adenocarcinoma.

13. A kit for determining a status of tumour absence/presence and/or of tumour regression/development, **characterised in that** it comprises:
- at least one compound which is capable of specifically binding to FADD proteins and/or to a phosphorylated p38-MAPKs, and/or at least one biological unit which is capable of producing such a compound, this at least one compound or biological unit being optionally contained in a physiological buffer, and/or optionally bound to a solid support, and
- appropriate instructions:
○ for using a FADD protein and/or phosphorylated p38-MAPK as cell indicator(s) the cellular presence/absence of which and/or the level of cellular expression of which is(are) correlated with a status of tumour absence/presence and/or of tumour regression/development, and/or
○ for using a FADD protein as an extracellular indicator the extracellular absence/presence of which and/or the level of extracellular presence of which is(are) correlated with a status of tumour absence/presence and/or of tumour regression/development.

14. An *in vitro* method for the prognosis of the resistance of a tumour to an anti-tumour chemotherapy, without having to implement said chemotherapy, **characterized in that** it comprises the following step:
- measuring the cellular amount of FADD proteins which is present in cells that are representative of the cells to which said anti-tumour chemotherapy is intended,
wherein said tumour is determined as resistant to said chemotherapy when said measured FADD cellular amount is significantly inferior to, and preferably twice as less the cellular amount of FADD proteins which is present in the cells of an immortalized FADD-expressing myeloid or lymphoid cell line, such as the THP1 cell line (ATCC TIB202) or the U937 cell line (ATCC CRL1593),
and/or the following step:
- measuring the amount of FADD proteins which is present in the extracellular environment of the cells to which said anti-myeloid tumour chemotherapy is intended,
wherein said tumour is determined as resistant to said chemotherapy when said measured extracellular FADD amount is at least twice as much the standard normal extracellular amount of FADD proteins which is observed in the extracellular environment of non-tumour but otherwise equivalent cells, provided that the standard normal cellular amount of FADD proteins which is observed in non-tumour but otherwise equivalent cells is significantly different from zero.

15. The *in vitro* method of claim 14, **characterized in that** said tumour is a myeloid tumour, such as a CML or an AML.

16. A kit for the prognosis of a status of resistance to an anti-tumour chemotherapy, **characterized in that** it comprises:
- at least one compound which is capable of specifically binding to FADD proteins, and/or at least one biological unit which is capable of producing such a compound, this at least one compound or biological unit being optionally contained in a physiological buffer, and/or optionally bound to a solid support, and
- appropriate instructions for using a FADD protein:
○ as a cell indicator the cellular absence of which and/or the level of cellular expression of which is(are) correlated with resistance to said anti-myeloid tumour chemotherapy, and/or
○ for using FADD proteins as an extracellular indicator the extracellular presence of which and/or the level of extracellular presence of which is(are) correlated with resistance to said anti-tumour chemotherapy.

17. A method to identify an anti-tumour active compound, **characterized in that** said anti-tumour active compound is selected by screening for a compound that prevents FADD proteins from being released from tumoural cells, or from *in vitro* cultured thyroid follicular cells.

18. An *in vitro* method to determine whether a compound can have an anti-tumour activity in an organism, without having to use any tumour-inducing agent, **characterised in that** it comprises the following steps:
- non-tumour thyroid follicular cells (TFC) are placed for *in vitro* growth under conditions of substrate composition, of temperature and of time which enable the adhesion and confluence of said TFC,
- said compound is placed in contact with said culture,
- at least one cellular amount selected from the group consisting of the cellular amount of FADD proteins and the cellular amount of phosphorylated p38-MAPKs is measured in said TFC after said contact with said compound,
wherein said compound is determined as capable of having an anti-tumour activity in said organism, when the measured cellular amount(s) is(are) either significantly superior to the (respective) control cellular amount(s) which is(are) observed in comparable *in* vitro-grown TFC but in the absence of contact with said compound, or not significantly different from the standard (respective) normal cellular amount(s) which is(are) measured in *ex vivo* non-tumour TFC,
and/or the following steps:
- non-tumour thyroid follicular cells (TFC) are placed for *in vitro* growth under conditions of substrate composition, of temperature and of time which enable the adhesion and confluence of said TFC,
- said compound is placed in contact with said culture,
- the extracellular amount of FADD proteins is measured in the extracellular environment of said TFC after said contact with said compound,
wherein said compound is determined as capable of having an anti-tumour activity in said organism, when the measured extracellular FADD amount is either significantly inferior to the control extracellular FADD amount which is observed in the extracellular environment of comparable *in* vitro-grown TFC but in the absence of contact with said compound, or not significantly different from the standard normal extracellular FADD amount which is measured in the extracellular environment of *ex vivo* non-tumour TFC.

19. The *in vitro* method according to claim 18, **characterised in that** said non-tumour TFC are submitted to a collagenase H treatment before being placed for *in vitro* growth.

20. The *in vitro* method according to claim 19 or 20, **characterized in that** said anti-tumour activity is an anti-adenoma or anti-adenocarcinoma activity.

21. A kit to determine in the absence of any tumour-inducing agent whether a compound can have an anti-tumour activity in an organism, **characterised in that** it comprises:
- non-tumour thyroid follicular cells (TFC), and
- at least one anti-FADD and/or anti-phospho-p38-MAPK compound.

22. An *in vitro* method to assess whether a compound has a high or a low probability of having a pro-tumour activity in an organism, **characterized in that** it comprises the following steps:
- said compound is placed *in vitro* into contact with cells of said organism, or with cells of a representative biological model thereof,
- at least one cellular amount selected from the group consisting of the cellular amount of FADD proteins and the cellular amount of phosphorylated p38-MAPKs is measured in said contacted cells, and
wherein said compound is determined as having a high probability of having a pro-tumour activity in said organism, when said measured cellular amount(s) is(are) significantly inferior to the standard (respective) normal cellular amount(s) which is(are) observed in non-tumour and uncontacted but otherwise equivalent cells,
wherein said compound is determined as having a low probability of having a pro-tumour activity in said organism, when said measured cellular amount(s) is(are) not significantly inferior to said standard (respective) normal cellular amount(s),
and/or the following steps:
- said compound is placed *in vitro* into contact with said cells of said organism, or with cells of a representative biological model thereof,
- the extracellular amount of FADD proteins which is present in the extracellular environment of the cells of said organism or representative biological model thereof is measured in said contacted cells,
wherein said compound is determined as having a high probability of having a pro-tumour activity in said organism, when said measured extracellular amount is significantly superior to the standard normal extracellular amount of FADD proteins which is observed in the extracellular environment of non-tumour and uncontacted but otherwise equivalent cells,
wherein said compound is determined as having a low probability of having a pro-tumour activity in said organism, when said measured extracellular amount is not significantly superior to said standard normal extracellular amount of FADD proteins,
provided that the standard normal cellular amount(s) of FADD proteins and/or of phosphorylated p38-MAPKs, respectively, which is(are) observed in non-tumour but otherwise equivalent cells, is(are) significantly different from zero.

23. The method of claim 22, **characterized in that** said pro-tumour activity is a pro-adenoma or pro-adenocarcinoma activity.

24. A kit to assess whether a compound has a high or a low probability of having a pro-tumour activity in an organism, **characterized in that** it comprises:
- at least one anti-FADD and/or anti-phospho-p38-MAPK specific compound, and optionally a tumour-free biological model, and
- appropriate instructions for using a FADD protein and/or a phosphorylated p38-MAPK as pro-tumour indicator(s).

25. *In vitro* use of a compound which is capable of specifically binding to FADD proteins and/or to phosphorylated p38-MAPKs, or of a biological unit which is capable of producing such a compound, for determining on a representative biological sample, whether there is or not a tumour in an organism, and/or for the prognosis of the resistance of a tumour to an anti-tumour chemotherapy, and/or for determining whether an applied anti-tumour treatment is or will be efficient or not in an organism, and/or for determining whether a compound can have an anti-tumour activity in an organism, and/or for assessing whether a compound has a high or a low probability of having a pro-tumour activity in an organism.
